(19) **Europäisches Patentamt / European Patent Office / Office européen des brevets**

(11) **EP 3 986 374 B1**

(12) **EUROPEAN PATENT SPECIFICATION**

(45) Date of publication and mention
of the grant of the patent:
**28.01.2026 Bulletin 2026/05**

(21) Application number: **20729684.9**

(22) Date of filing: **27.05.2020**

(51) International Patent Classification (IPC):
**A61K 9/1271** *(2025.01)* **A61K 9/00** *(2006.01)*
**A61K 9/51** *(2006.01)* **A61K 38/26** *(2006.01)*
**A61P 3/10** *(2006.01)*

(52) Cooperative Patent Classification (CPC):
**A61K 9/1271; A61K 9/51; A61K 38/26; A61P 3/10**

(86) International application number:
**PCT/EP2020/064766**

(87) International publication number:
**WO 2020/254083 (24.12.2020 Gazette 2020/52)**

(54) **LIPID NANOCAPSULES CHARGED WITH INCRETIN MIMETICS**

MIT INKRETINMIMETIKA GELADENE LIPIDNANOKAPSELN

NANOCAPSULES LIPIDIQUES CHARGÉES EN MIMÉTIQUES D'INCRÉTINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.06.2019 US 201962864661 P
23.07.2019 EP 19187820**

(43) Date of publication of application:
**27.04.2022 Bulletin 2022/17**

(73) Proprietor: **Université catholique de Louvain
1348 Louvain-la-Neuve (BE)**

(72) Inventors:
• **BELOQUI, Ana
1000 Bruxelles (BE)**
• **XU, Yining
1200 Woluwe-Saint-Lambert (BE)**
• **PRÉAT, Véronique
1950 Kraainem (BE)**
• **CANI, Patrice
1200 Bruxelles (BE)**

(74) Representative: **Icosa
83 avenue Denfert-Rochereau
75014 Paris (FR)**

(56) References cited:
**WO-A1-2018/157202    US-A1- 2017 087 096**

• NEHA SHRESTHA ET AL: "The stimulation of GLP-1 secretion and delivery of GLP-1 agonists via nanostructured lipid carriers", NANOSCALE, vol. 10, no. 2, 1 January 2018 (2018-01-01), United Kingdom, pages 603 - 613, XP055662639, ISSN: 2040-3364, DOI: 10.1039/C7NR07736J
• XU YINING ET AL: "Size Effect on Lipid Nanocapsule-Mediated GLP-1 Secretion from Enteroendocrine L Cells.", MOLECULAR PHARMACEUTICS 02 01 2018, vol. 15, no. 1, 2 January 2018 (2018-01-02), pages 108 - 115, XP002797234, ISSN: 1543-8392
• ANTON N ET AL: "Reverse micelle-loaded lipid nano-emulsions: New technology for nano-encapsulation of hydrophilic materials", INTERNATIONAL JOURNAL OF PHARMACEUTICS, ELSEVIER, NL, vol. 398, no. 1-2, 15 October 2010 (2010-10-15), pages 204 - 209, XP027261267, ISSN: 0378-5173, [retrieved on 20100902]

Remarks:
The complete document including Reference Table(s) and the Sequence Listing(s) can be downloaded from the EPO website

**EP 3 986 374 B1**

**Description**

**FIELD OF INVENTION**

The present invention relates to the field of lipid nanocapsule-based drug delivery systems and their use for treating GLP-1-associated disorders.

**BACKGROUND OF INVENTION**

The development of oral dosage forms that enable the absorption of therapeutic peptides into the systemic circulation is one of the greatest challenges for the pharmaceutical industry. Selected diabetes peptides have transitioned to the late phase of development despite their low oral bioavailability (estimated to be between 0.5-1.0%). Nevertheless, the benefits of oral peptide delivery over an intravenous or subcutaneous administration are remarkable, and especially in the case of anti-diabetic drugs, e.g., glucagon-like peptide-1 (GLP-1). The oral administration of incretin mimetic peptides has the additional therapeutic advantage of simulating the normal physiological pathway of the native peptide. GLP-1 agonists target the hepatoportal zone, which can be accessed in much higher concentrations via the hepatic portal vein than via subcutaneous delivery, thereby reducing systemic exposure and its associated side effects. Despite the numerous ongoing efforts to exploit the oral route of administration of incretin mimetic peptides, there is only one GLP-1 analog currently administered orally in the market (semaglutide, Rybelsus®, developed by NovoNordisk), which requires coadministration of a functional excipient, namely sodium N-[8-(2-hydroxybenzoyl) amino] caprylate (SNAC).

The gut physiology offers a stimulating environment, which has not yet been fully exploited to its full potential in the drug delivery field. A wide variety of cells are scattered through the gastrointestinal epithelium. Among these, enteroendocrine L cells have attracted particular interest because of the pleiotropic effects of their secreted peptides (*e.g.,* GLP-1 and GLP-2). These cells have a relatively rapid turnover of 5-7 days, and their density is increased in pathological conditions, such as type 2 diabetes mellitus (T2DM) and inflammatory bowel diseases (IBD), making them attractive targets for the treatment of these diseases. In the context of T2DM, GLP-1 secreted from intestinal L cells stimulates postprandial insulin secretion and is quickly hydrolyzed by dipeptidyl peptidase-IV (DPP-IV). Thus, several GLP-1 analogs with improved plasma half-life (e.g., exenatide, liraglutide, semaglutide) have been developed and proven to be successful for treating T2DM. Recently, researchers have turned their attention from the secreted peptides towards the L cells themselves as targets for the treatment of obesity, T2DM, and IBD. Indeed, enhancing endogenous GLP-1 secretion would represent a more physiological and novel alternative in incretin-based diabetes therapy (Burant, Diabetes Care. 2013; 36 Suppl 2, S175-179). While certain endogenous ligands found in the gut lumen, such as short chain fatty acids that include butyrate and propionate, can activate L cells, the use of nanocarriers could represent an alternative therapeutic strategy to stimulate the production of gut peptides. Indeed, nanocarriers could be engineered to simulate certain ligands and can be designed to have increased gastrointestinal retention, thereby evoking long-term activation of L cells.

Current strategies for oral peptide delivery use delivery systems merely as a vehicle. Examples of delivery systems are found in the prior art, such as in US2017/087096 A1, Sherestha et al. (2018, Nanoscale, 10(2): 603-613) or Anton et al. (2010, Int. J. of Pharmaceutics, Elsevier, NL, 398(1-2):204-209). None of them exploit the physiological properties of the carrier itself to achieve maximal therapeutic potential of the formulation.

The inventors have developed an unprecedented approach using incretin mimetics via the oral route to treat and/or prevent metabolic disorders associated with dysfunctional glycemia. Indeed, they provide evidences that combining nanocarriers with GLP-1 analogs is sufficient to normalize the glycemia of obese/diabetic mice after either acute or chronic treatment. The lipid nanocapsule-based drug delivery system of the present invention synergizes its own biological effect (stimulation of GLP-1 release) and that of the encapsulated bioactive molecule (incretin mimetic). Interestingly, in addition to the strong advantage of using the oral route, this approach is at least as efficient as the current marketed drug and could even be more potent for improving oral glucose tolerance, insulin resistance, lipid distribution and hepatic steatosis. Thus, this strategy offers an additional advantage over current approaches for oral incretin mimetic peptide delivery, providing increased endogenous GLP-1 levels.

**SUMMARY**

The present invention relates to a lipid nanocapsule for oral administration comprising:

- a solid lipid outer shell; and
- a lipophilic liquid inner core comprising reverse micelles loaded with one or more incretin mimetics.

In one embodiment, the solid lipid outer shell comprises one or more surfactants selected in a group comprising ionic surfactants, nonionic surfactants, amphoteric surfactants, lipophilic surfactants, and mixtures thereof.

In one embodiment, the lipophilic liquid inner core comprises one or more oils. In one embodiment, the one or more oils is a triglyceride, a fatty acid, a fatty acid ester or a mixture thereof.

In one embodiment, the reverse micelles comprise one or more ingredients selected in a group comprising a surfactant, an oil and a mixture thereof.

In one embodiment, the lipid nanocapsule has a mean diameter ranging from about 100 nm to about 300 nm.

In one embodiment, the lipid nanocapsule induces endogenous GLP-1 secretion *in vivo*.

In one embodiment, the one or more incretin mimetics is selected in a group comprising albiglutide, dulaglutide, exenatide, liraglutide, lixisenatide and semaglutide. In one embodiment, the one or more incretin mimetics is exenatide.

In one embodiment, the nanocapsule comprises:

- a solid lipid outer shell comprising a poly-oxyethylene ester of fatty acid, a non-ionic lipophilic surfactant and optionally a PEGylated lipid, in particular a PEGylated phospholipid; and
- a lipophilic liquid inner core comprising a triglyceride and a fatty acid ester, and comprising reverse micelles loaded with exenatide.

In one embodiment, the lipid nanocacpsule comprises:

- a solid lipid outer shell comprising the surfactants Solutol® HS15, Lipoid® S100 and optionally DSPE-PEG$_{2000}$-OCH$_3$; and

- a lipophilic liquid inner core comprising the oils Labrafac™ Lipophile WL1349 and/or Plurol® CC 497 or Peceol™, and comprising reverse micelles loaded with exenatide.

In one embodiment, the exenatide has a pharmacokinetic profile characterized in a relative bioavailability of at least 4% in obese/diabetic high fat diet-induced mice.

Another object of the invention is a pharmaceutical composition comprising:

- a therapeutically effective amount of a lipid nanocapsule for oral administration comprising a solid lipid outer shell, and a lipophilic liquid inner core comprising reverse micelles loaded with one or more incretin mimetics, and
- a pharmaceutically acceptable vehicle.

The invention also relates to a medicament comprising a therapeutically effective amount of a lipid nanocapsule for oral administration comprising a solid lipid outer shell and a lipophilic liquid inner core comprising reverse micelles loaded with one or more incretin mimetics.

The invention further relates to a lipid nanocapsule comprising a solid lipid outer shell and a lipophilic liquid inner core comprising reverse micelles loaded with one or more incretin mimetics for use in treating and/or preventing a disorder associated with a GLP-1 dysfunction in a subject in need thereof.

In one embodiment, the disorder is selected in a group comprising type 2 diabetes mellitus (T2DM), obesity, inflammatory bowel disease (IBD), pancreatitis, dyslipidemia, non-alcoholic fatty liver disease, hyperglycemia, liver steatosis, overweight, nonalcoholic fatty liver disease (NAFLD), nonalcoholic steatohepatitis (NASH), insulin resistance, hyperinsulinemia, glucose intolerance, hyperglycemia, metabolic syndrome, prediabetes, impaired fasting glucose, hyperphagia, altered food intake behaviour, hepatic insulin resistance, whole body insulin resistance, accelerated transit, adipose tissue inflammation, cardiac dysfunctions, acute myocardial infarction, hypertension, cardiovascular disease, atherosclerosis, peripheral arterial disease, stroke, heart failure, coronary heart disease, kidney disease, diabetic complications, neuropathy, and gastroparesis. In one embodiment, said disorder is selected in a group comprising type 2 diabetes mellitus (T2DM), obesity and inflammatory bowel disease (IBD).

Another object of the invention is a kit for treating and/or preventing a disorder associated with a GLP-1 dysfunction comprising:

- one or more lipid nanocapsules comprising a solid lipid outer shell, and a lipophilic liquid inner core comprising reverse micelles loaded with one or more incretin mimetics; and
- one or more oral hypoglycemic agent.

**DEFINITIONS**

In the present invention, the following terms have the following meanings:

- **"About"** preceding a value means plus or less 10% of said value. It is to be understood that the value to which the term "about" refers to is itself also specifically, and preferably, disclosed.

- **"Comprise"** is intended to mean **"contain", "encompass"** and **"include".** In some embodiments, the term **"comprise"** also encompasses the term **"consist** of".

- **"Pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. A pharmaceutically acceptable carrier or excipient refers to a non-toxic solid, semi-solid or liquid filler, diluent, encapsulating material or formulation auxiliary of any type. For human administration, preparations should meet sterility, general safety and purity standards as required by FDA Office of Biologics standards.

- **"Subject"** refers to a mammal, preferably a human. In one embodiment, the subject is a man. In another embodiment, the subject is a woman. In one embodiment, a subject may be a "patient", i.e. a warm-blooded animal, more preferably a human, who/which is awaiting the receipt of, or is receiving medical care or was/is/will be the object of a medical procedure, or is monitored for the development of GLP-1-associated disorder. In one embodiment, the subject is an adult (for example a subject above the age of 18). In another embodiment, the subject is a child (for example a subject below the age of 18).

- **"Therapeutically effective amount"** means the level or amount of agent that is aimed at, without causing significant negative or adverse side effects to the target, (1) delaying or preventing the onset of GLP-1-associated disorder; (2) slowing down or stopping the progression, aggravation, or deterioration of one or more symptoms of GLP-1-associated disorder; (3) bringing about ameliorations of the symptoms of GLP-1-associated disorder; (4) reducing the severity or incidence of GLP-1-associated disorder; or (5) preventing GLP-1-associated disorder. In one embodiment, a therapeutically effective amount is administered prior to the onset of GLP-1-associated disorder, for a prophylactic or preventive action. In another embodiment, a therapeutically effective amount is administered after the onset of GLP-1-associated disorder, for a therapeutic action.

- **"Treatment"** refers to both therapeutic treatment and prophylactic or preventative measures wherein the object is to prevent or slow down (lessen) the GLP-1-associated disorder. Those in need of treatment include those already with the disorder as well as those prone to have the disorder or those in whom the disorder is to be prevented. A subject or mammal is successfully "treated" if, after receiving a therapeutic amount of lipid nanocapsules according to the present invention, the subject or mammal shows one or more of the following observable and/or measurable changes: amelioration related to one or more of the symptoms associated with the GLP-1-associated disorder, reduction of morbidity and mortality and improvement in quality of life issues. The above parameters for assessing successful treatment and improvement in the disease are readily measurable by routine procedures familiar to a physician.

## DETAILED DESCRIPTION

The inventors have formulated incretin mimetics loaded lipid nanocapsules for oral administration, for the purpose of treating and/or preventing disorders associated with a GLP-1 dysfunction, such as, *e.g.,* type 2 diabetes mellitus (T2DM), obesity. The nanocapsules according to the invention structurally differ notably from the lipid-based formulations disclosed by, e.g., US2017087096 and by WO2018157202, and from the nanoparticles disclosed by Shrestha et al. (Nanoscale. 2018; 10:603-613) and Beloqui et al. (Mol Pharm. 2016; 13:4222-4230). In addition, as evidenced by comparative study disclosed herein (see comparative example 3), these nanoparticles from the state in the art are not capable to induce GLP-1 secretion in further studies *in vivo,* nor decrease hyperglycemia and hyperinsulinemia, whereas the nanocapsules according to the invention are. In addition, PEGylated nanocapsules according to the invention further improve the half-life or the mimetic incretin, as well as its systemic absorption.

This invention relates to a lipid nanocapsule for oral administration comprising:

- a solid lipid outer shell; and
- a lipophilic liquid inner core comprising reverse micelles loaded with one or more incretin mimetics.

In some embodiments, the ingredients used to prepare the lipid nanocapsules according to the instant invention are belonging to the list of "generally recognized as safe" (GRAS) ingredients.

As used herein, the terms "nanocapsule" and "nanoparticle" may be substituted one with the other.

According to a first embodiment, the solid lipid outer shell of the lipid nanocapsule according to the invention comprises one or more (or at least one) surfactant.

In one embodiment, the one or more surfactant is PEGylated.

In one embodiment, the Hydrophilic-Lipophilic Balance (HLB) of the at least one surfactant is ranging from about 4 to about 40, preferably from about 6 to about 16, more preferably from about 10 to about 14.

The HLB value is defined by C. Larpent in Traité K.342 of the Editions Techniques de 1'Ingenieur.

In one embodiment, the at least one surfactant is an ionic, a nonionic or an amphoteric surfactant. In one embodiment, the at least one surfactant is selected in a group comprising ethoxylated fatty alcohols, ethoxylated fatty acids, partial glycerides of ethoxylated fatty acids and polyethoxylated fatty acid triglycerides, and mixtures thereof. In a particular embodiment, the at least one surfactant, in particular non-ionic surfactant, is a poly-oxyethylene ester of fatty acid, preferably Solutol® HS15 (also named Kolliphor® HS 15).

Examples of ethoxylated fatty alcohols include, but are not limited to, adducts of ethylene oxide with lauryl alcohol, especially those comprising from 9 to 50 oxyethylene groups (Laureth-9 to Laureth-50 in CTFA names); adducts of ethylene oxide with behenyl alcohol, especially those comprising from 9 to 50 oxyethylene groups (Beheneth-9 to Beheneth-50 in CTFA names); adducts of ethylene oxide with cetostearyl alcohol (mixture of cetyl alcohol and stearyl alcohol), especially those comprising from 9 to 30 oxyethylene groups (Ceteareth-9 to Ceteareth-30 in CTFA names); adducts of ethylene oxide with cetyl alcohol, especially those comprising from 9 to 30 oxyethylene groups (Ceteth-9 to Ceteth-30 in CTFA names); adducts of ethylene oxide with stearyl alcohol, especially those comprising from 9 to 30 oxyethylene groups (Steareth-9 to Ceteareth-30 in CTFA names); adducts of ethylene oxide with isostearyl alcohol, especially those comprising from 9 to 50 oxyethylene groups (Isosteareth-9 to Isosteareth-50 in CTFA names); and mixtures thereof.

Examples of ethoxylated fatty acids include, but are not limited to, adducts of ethylene oxide with lauric, palmitic, stearic or behenic acid, and mixtures thereof, especially those comprising from 9 to 50 oxyethylene groups, such as PEG-9 to PEG-50 laurates (CTFA names: PEG-9 laurate to PEG-50 laurate); PEG-9 to PEG-50 palmitates (CTFA names: PEG-9 palmitate to PEG-50 palmitate); PEG-9 to PEG-50 stearates (CTFA names: PEG-9 stearate to PEG-50 stearate); PEG-9 to PEG-50 palmitostearates; PEG-9 to PEG-50 behenates (CTFA names: PEG-9 behenate to PEG-50 behenate); and mixtures thereof.

In one embodiment, the at least one surfactant is a heat-sensitive, hydrophilic, nonionic surfactant.

In one embodiment, the solid lipid outer shell of the lipid nanocapsule according to the invention further comprises at least one lipophilic surfactant.

In one embodiment, the at least one lipophilic surfactant is a phospholipid. In one embodiment, the at least one surfactant is selected in a group comprising phosphatidylcholines (also named lecithin), phosphatidylglycerol, phosphatidylinositol, phosphatidylserine, phosphatidic acid and phosphatidylethanolamine. Examples of lipophilic surfactants include, but are not limited to, Lipoid S 45, Lipoid S 75-3, Lipoid S 100, Lipoid GPC, Lipoid E 80, sorbitan oleate (Span 80), Epikuron™135 and Plurol®. In a particular embodiment, the lipophilic surfactant is Lipoid S 100 and/or sorbitan oleate (Span 80).

In one embodiment, the at least one surfactant, in particular a lipophilic surfactant, more particularly a lipid, more particularly a phospholipid, is a PEGylated lipid, more particularly a PEGylated phospholipid. Examples of PEGylated phospholipids include, but are not limited to, DPPE-PEGx (wherein DPPE stands for dipalmitoylphosphatidylethanolamine), DSPE-PEGx (wherein DSPE stands for distearoylphosphatidylethanolamine), DOPE-PEGx (wherein DOPE stands for dioleoylphosphatidylethanolamine) and POPE-PEGx (wherein POPE stands for palmitoyloleylphosphatidylethanolamine), in which x represents the size of the PEG molecule in g/mol. In some embodiments, x is comprised from about 400 to about 20,000, preferably from about 800 to about 5,000, more preferably from about 1,000 to about 3,000. As used herein, from about 400 to about 20,000 includes about 400, 500, 600, 700, 800, 900, 1,000, 2,000, 3,000, 4,000, 5,000, 6,000, 7,000, 8,000, 9,000, 10,000, 11,000, 12,000, 13,000, 14,000, 15,000, 16,000, 17,000, 18,000, 19,000 and 20,000. In one embodiment, x is 1,000, 2,000, 3,000, 4,000, or 5,000. In one embodiment, x is 6,000, 7,000, 8,000, or 9,000. In one embodiment, x is 10,000.

As used herein, the term "PEG" refers to a polyethylene glycol compound of the general formula (I), as commonly accepted in the state of the art:

$$\text{H-[O-CH}_2\text{-CH}_2\text{]x-R} \qquad \text{(I)},$$

wherein x is comprised from about 400 to about 20,000 (as being representative of the size of the PEG molecule in g/mol) and wherein R represents -OH, a -O(C$_1$-C$_{12}$) alkoxyl group, a -(C$_1$-C$_{12}$) carboxylic group, -NH$_2$.

In one embodiment, R represents a -O(C$_1$-C$_6$) alkoxyl group including -OCH$_3$ (methoxyl), -OC$_2$H$_5$, -OC$_3$H$_7$, -OC$_4$H$_9$, -OC$_5$H$_{11}$, -OC$_6$H$_{13}$ and isomers thereof. In one embodiment, R represents a -(C$_1$-C$_6$) carboxylic group including -COOH (methanoic acid), -CH$_2$-COOH (ethanoic acid), -C$_2$H$_4$-COOH (propionic acid), -C$_3$H$_6$-COOH (butyric acid), -C$_4$H$_8$-COOH (pentoic acid), -C$_5$H$_{10}$-COOH (hexanoic acid) and isomers thereof.

In one embodiment, the PEGylated phospholipid is DSPE-PEG$_{2000}$-OCH$_3$ (1,2-distearoyl-sn-glycero-3-phosphoethanolamine-methoxylpoly(ethylene glycol)2000), which is a DSPE-PEG comprising a PEG of formula (I), wherein x is 2,000 and R is -OCH$_3$.

In one embodiment, the PEGylated phospholipid is DSPE-PEG$_{2000}$-CH$_2$-CH$_2$-COOH (1,2-distearoyl-sn-glycero-3-phosphoethanolamine-N-[poly(ethylene glycol)-2000]-propionate), which is a DSPE-PEG comprising a PEG of formula (I), wherein x is 2,000 and R is propionic acid).

In practice, PEGylated phospholipids provide with enhance mucus diffusion, increased stability and prolonged blood circulation. An increased mucopenetration can help increase the contact of the nanocapsules with the surface of the L cells. In practice, PEGylated phospholipids may be commercially available from, e.g., Nanocs® or Nanosoft Polymers®.

In one embodiment, the lipid outer shell of the lipid nanocapsule according to the invention comprises one or more ingredients selected in a group comprising a nonionic surfactant, a lipophilic surfactant and a mixture thereof.

In one embodiment, the lipid outer shell of the lipid nanocapsule according to the invention comprises one or more ingredients selected in a group comprising a heat-sensitive hydrophilic nonionic surfactant, a phospholipid and a mixture thereof. In one embodiment, the solid lipid outer shell of the lipid nanocapsule according to the invention comprises a heat-sensitive hydrophilic nonionic surfactant and a phospholipid.

In one embodiment, the lipid outer shell of the lipid nanocapsule according to the invention comprises one or more surfactants, preferably Lipoid® S100 and/or Solutol® HS15, in a total amount ranging from about 1% to 40% by weight, preferably from about 3% to about 25% by weight, more preferably from about 5% to about 15% by weight, with respect to the total weight of the lipid nanocapsule. In one embodiment, the lipid outer shell of the lipid nanocapsule according to the invention comprises one or more surfactants, preferably Lipoid® S100 and/or Solutol® HS15, in a total amount of about 6.45% by weight, with respect to the total weight of the lipid nanocapsule.

In one embodiment, the lipid outer shell of the lipid nanocapsule according to the invention comprises one or more surfactants, preferably Lipoid® S100 and/or Solutol® HS15 and/or DSPE-PEG$_{2000}$-OCH$_3$ or DSPE-PEG$_{2000}$-CH$_2$-CH$_2$-COOH, in a total amount ranging from about 0.1% to 40% by weight, preferably from about 0.5% to about 25% by weight, more preferably from about 1% to about 10% by weight, with respect to the total weight of the lipid nanocapsule.

Within the scope of the instant invention, the expression "from about 0.1% to about 40% by weight" encompasses 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% and 40% by weight.

In one embodiment, the lipid outer shell of the lipid nanocapsule according to the invention comprises one or more non-ionic surfactants, preferably Solutol® HS15, and one or more lipophilic surfactants, preferably Lipoid® S100 and/or a PEGylated phospholipid. In one embodiment, the lipid outer shell of the lipid nanocapsule according to the invention comprises one non-ionic surfactant, preferably Solutol® HS15, and 2 lipophilic surfactants, preferably Lipoid® S100 and a PEGylated phospholipid.

In one embodiment, the lipophilic liquid inner core of the lipid nanocapsule according to the invention comprises one or more oils.

In one embodiment, the oil is at least one triglyceride, a fatty acid, a fatty acid ester or a mixture thereof.

In one embodiment, the fatty acid is selected from saturated or unsaturated C$_8$ to C$_{26}$ fatty acids, and mixtures thereof. In one embodiment, the unsaturated fatty acid may be a monounsaturated or a polyunsaturated fatty acid. In one embodiment, the fatty acid is polyunsaturated, in particular is selected from the group of omega-3 fatty acids, which comprises alpha-linolenic acid (18:3, ALA), eicosapentaenoic acid (20:5, EPA) and docosahexaenoic acid (22:6, DHA). In practice a source of omega-3 fatty acid may be fish oil.

In one embodiment, the fatty acid ester is selected from C$_8$ to C$_{18}$, preferably from C$_8$ to C$_{12}$, fatty acid esters. In a particular embodiment, the fatty acid ester is selected in a group comprising or consisting of ethyl palmitate, ethyl oleate, ethyl myristate, isopropyl myristate, octyldodecyl myristate, and mixtures thereof.

In one embodiment, the triglyceride is a synthetic triglyceride or a triglyceride of natural origin. The natural sources of triglycerides include, but are not limited to, animal fats or plant oils, for example soybean oils or sources of long-chain triglycerides (LCT).

In another embodiment, the triglyceride is composed from medium-length fatty acids, also known as medium-chain triglycerides (MCT). A medium-chain triglyceride (MCT) oil is a triglyceride in which the hydrocarbon chain contains from 8 to 12 carbon atoms.

Examples of MCT oils include, but are not limited to, TCR products (commercial name from the Société Industrielle des Oléagineux, France, for a triglyceride mixture in which about 95% of the fatty acid chains contain 8 or 10 carbon atoms) and Myglyol® 812 (triglyceride sold by the company Dynamit Nobel, Sweden, for a mixture of caprylic and capric acid glyceride triesters).

In one embodiment, the fatty acid units of the triglycerides are unsaturated, monounsaturated or polyunsaturated. In one embodiment, the lipophilic liquid inner core comprises a mixture of triglycerides containing variable fatty acid units.

In one embodiment, the oil is selected in a group comprising a glycerol, glyceryl fatty acid monoglyceride, fatty acid triglycerides, mono-, di- and triglycerides of linoleic and/or oleic acids, caprylic acid triglyceride, capric acid triglyceride, propylene glycol esters of caprylic and/or capric acid, triglycerides of plant fatty acids, and a mixture thereof.

Examples of oils that may be comprised in the lipophilic inner core include, but are not limited to, Labrafac™ Lipophile WL1349 (medium-chain triglycerides of caprylic and capric acids), Labrafac™ PG (propylene glycol esters of caprylic and capric acids), Plurol® CC 497 (polyglyceryl-6 dioleate). Peceol™ (Glyceryl monooleate), Maisine® CC (mono-, di- and triglycerides of mainly linoleic and oleic acids), and Miglyol® 810/812 (triglyceride of caprylic and capric acids). In a particular embodiment, the lipophilic inner core comprises Labrafac™ Lipophile WL 1349. In a particular embodiment, the lipophilic inner core comprises Plurol® CC 497 or Peceol™. As used herein, the terms "Labrafac™ Lipophile WL1349" and "Labrafac® WL 1349" may be used indifferently and refer to the same ingredient.

In one embodiment, the lipophilic inner core of the lipid nanocapsule according to the invention comprises one or more oils, preferably Labrafac™ Lipophile WL1349 and/or Peceol® (or Plurol® CC 497), in a total amount ranging from about 25% to about 65% by weight, preferably from about 30% to about 55% by weight, more preferably from about 35% to about 45% by weight with respect to the total weight of the lipid nanocapsule. In one embodiment, the lipophilic inner core of the lipid nanocapsule according to the invention comprises one or more oils, preferably Labrafac™ Lipophile WL1349 and/or Peceol® (or Plurol® CC 497), in a total amount of about 41.4% weight with respect to the total weight of the lipid nanocapsule.

Within the scope of the instant invention, the expression "from about 25% to about 65% by weight" encompasses 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, and 65% by weight.

In one embodiment, the lipid nanocapsules of the invention further comprise at least one salt and water. In one embodiment, the lipid nanocapsules of the invention further comprise sodium chloride and water.

In one embodiment, the aqueous phase in which the lipid nanocapsules of the invention are prepared, initially contains a certain concentration of salts, preferably sodium chloride (NaCl). In one embodiment, this concentration of salts is ranging from about 2% to about 8% by weight, preferably from about 4% to about 6% by weight, more preferably from about 4.2% to about 5.2% by weight with respect to the total weight of the lipid nanocapsule.

Within the scope of the invention, the expression "from about 2% to about 8%" encompasses 2.0%, 2.5%, 3.0%, 3.5%, 4.0%, 4.5%, 5.0%, 5.5%, 6.0%, 6.5%, 7.0%, 7.5% and 8.0% by weight.

In one embodiment, the aqueous phase in which the lipid nanocapsules of the invention are prepared further comprises osmotic agents, cryoprotective agents, lyoprotective agents, preserving agents or mixtures thereof. In one embodiment, cryoprotective or lyoprotective agents are mannitol or trehalose. In one embodiment, cryoprotective or lyoprotective agents are added to the suspension of nanocapsules to be lyophilized.

In one embodiment, the reverse micelles of the lipid nanocapsule according to the invention comprise one or more ingredients selected in a group comprising a surfactant, an oil and a mixture thereof.

In one embodiment, the surfactant of the reverse micelles has an HLB value of less or equal to 10, preferably of less than or equal to 8, more preferably of less than or equal to 6.

In one embodiment, the surfactant of the reverse micelles is a fatty ester. In one embodiment, the surfactant of the reverse micelles is a sorbitan oleate, a sorbitan monolaurate, a sorbitan trioleate, a sorbitan monooleate, a polyoxyethylene of sorbitan monooleate, a polyoxyethylene of sorbitan trioleate, or a mixture thereof. In a particular embodiment, the surfactant of the reverse micelles is Span® 80, Span® 20, Span® 85, Tween® 20, Tween® 80 or Tween® 85.

In one embodiment, the oil of the reverse micelles comprises phospholipids. In one embodiment, the phospholipids include phosphatidylcholine, phosphatidylglycerol, phosphatidylinositol, phosphatidylserine, phosphatidic acid and phosphatidylethanolamine, and a mixture thereof.

In one embodiment, the oil of the reverse micelles comprises fatty acid triglycerides, preferably caprylic acid triglyceride and/or capric acid triglyceride or a mixture thereof. In a particular embodiment, the oil of the reverse micelles comprises Labrafac™ Lipophile WL1349.

In one embodiment, the reverse micelles of the lipid nanocapsule according to the invention comprise a surfactant and an oil as described hereinabove.

In a particular embodiment, the reverse micelles of the lipid nanocapsule according to the invention comprise or consist of at least one incretin mimetic, Span® 80 and Labrafac™ Lipophile WL1349. In a particular embodiment, the reverse micelles of the lipid nanocapsule according to the invention comprise or consist of exenatide, Span® 80 and Labrafac™ Lipophile WL1349.

In one embodiment, the reverse micelles of the invention comprise from about 5% to about 50% by weight of surfactant, preferably Span® 80, preferably from about 10% to about 35% by weight, more preferably from about 15% to about 20% by weight, with respect to the total weight of the reverse micelles. In a particular embodiment, the reverse micelles of the invention comprise about 16.67% by weight of surfactant, with respect to the total weight of the reverse micelles, preferably Span® 80.

Within the scope of the invention, the expression "from about 5% to about 50% by weight" encompasses 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% and 50% by weight.

In one embodiment, the reverse micelles of the invention comprise from about 50% to about 95% by weight of oil, preferably Labrafac™ Lipophile WL1349, preferably from about 75% to about 90% by weight, more preferably from about 80% to about 85% by weight, with respect to the total weight of the reverse micelles. In a particular embodiment, the reverse micelles of the invention comprise about 83.33% of oil by weight, with respect to the total weight of the reverse micelles, preferably Labrafac™ Lipophile WL1349.

Within the scope of the invention, the expression "from about 50% to about 95% by weight" encompasses 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94% and 95% by weight.

In one embodiment, the reverse micelles represent from about 0.01% to about 40% by weight, preferably from about 1% to about 35% by weight, more preferably from about 10% to about 25% by weight, with respect to the total weight of the lipid nanocapsules.

Within the scope of the invention, the expression "from about 0.01% to about 40% by weight" encompasses 0.01%, 0.05%, 0.1%, 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% and 40% by weight.

In one embodiment, the surfactant/oil weight ratio is ranging from 1:10 to 1:1. In one embodiment, the surfactant/oil weight ratio is ranging from 1:8 to 1:3. In one embodiment, the surfactant/oil weight ratio is 1:5. Within the scope of the invention, the expression "from 1:10 to 1:1" encompasses 1:10, 1:9, 1:8, 1:7, 1:6, 1:5, 1:4, 1:3, 1:2 and 1:1.

In one embodiment, the incretin mimetics/surfactant(s) weight ratio is ranging from 0.001 to 0.2, preferably from 0.005 to 0.05, more preferably from 0.01 to 0.03. In one embodiment, the incretin mimetics/surfactant(s) weight ratio is about 0.03. Within the scope of the invention, the expression "from 0.001 to 0.2" encompasses 0.001, 0.0025, 0.005, 0.0075, 0.01, 0.025, 0.05, 0.075, 0.1, 0.125, 0.15, 0.175 and 0.2.

In one embodiment, the lipid nanocapsule according to the invention has a mean diameter of at least about 100, 110, 120, 125, 130, 140, 150, 160, 170, 175, 180, 190 or 200 nm. In one embodiment, the lipid nanocapsule according to the invention has a mean diameter of at most about 200, 210, 220, 230, 240, 250, 260, 270, 280, 290 or 300 nm.

In one embodiment, the lipid nanocapsule according to the invention has a mean diameter ranging from about 100 nm to about 300 nm, preferably from about 150 nm to about 250 nm, more preferably from about 180 nm to about 230 nm. In one embodiment, the lipid nanocapsule according to the invention has a mean diameter ranging from about 200 nm to about 300 nm. In a particular embodiment, the lipid nanocapsule according to the invention has a mean diameter of about 200 nm. In practice, lipid nanocapsules having a mean diameter above about 200 nm have the capacity to induce the secretion of endogenous GLP-1 and hence have the capacity to promote an increase of the GLP-1 blood level. In some embodiments, the lipid nanocapsule according to the invention induces endogenous GLP-1 secretion *in vivo.* In practice, the secretion of endogenous GLP-1 *in vivo* may be measured according to any suitable method from the state of the art, or a method adapted therefrom. Illustratively, the level of GLP-1 may be measured in a blood sample, in particular a plasma sample, by the mean of an ELISA kit, such as, *e.g.,* the commercial Total GLP-1 ELISA kit, (from Meso Scale Delivery®). The endogenous GLP-1 secretion may be expressed as the fold-change compared with a reference value acquired before treatment.

In one embodiment, the incretin mimetics of the lipid nanocapsule according to the invention are selected in a group comprising exenatide, albiglutide, dulaglutide, liraglutide, lixisenatide and semaglutide.

In one embodiment, the lipid nanocapsule according to the invention comprises at least exenatide.

In one embodiment, exenatide has a pharmacokinetic profile characterized in a relative bioavailability of at least 4% in obese/diabetic high fat diet-induced mice.

Within the scope of the invention, the expression "at least 4%" includes 4%, 5%, 6%, 7%, 8%, 9%, 10%, 11%, 12%, 15%, or more.

In a particular embodiment, the lipid nanocapsule according to the invention comprises:

- a solid lipid outer shell; and
- a lipophilic liquid inner core comprising comprising reverse micelles loaded with exenatide.

In a particular embodiment, the lipid nanocapsule according to the invention comprises:

- a solid lipid outer shell comprising a poly-oxyethylene ester of fatty acid and a lipophilic surfactant; and
- a lipophilic liquid inner core comprising a triglyceride and a fatty acid ester, and comprising reverse micelles loaded with exenatide.

In a particular embodiment, the lipid nanocapsule according to the invention comprises:

- a solid lipid outer shell comprising the surfactants Solutol® HS15 and Lipoid® S100; and
- a lipophilic liquid inner core comprising the oils Labrafac™ Lipophile WL1349 and/or Plurol® CC 497 or Peceol™, and comprising reverse micelles loaded with exenatide.

In a particular embodiment, the lipid nanocapsule according to the invention consists of:

- a solid lipid outer shell consisting of the surfactants Solutol® HS15 and Lipoid® S100; and
- a lipophilic liquid inner core consisting of the oils Labrafac™ Lipophile WL1349 and/or Plurol® CC 497 or Peceol™, sodium chloride, water and reverse micelles loaded with exenatide.

In a particular embodiment, the lipid nanocapsule according to the invention comprises:

- a solid lipid outer shell comprising a poly-oxyethylene ester of fatty acid, a non-ionic lipophilic surfactant and optionally a PEGylated lipid, in particular a PEGylated phospholipid; and
- a lipophilic liquid inner core comprising a triglyceride and a fatty acid ester, and comprising reverse micelles loaded with exenatide.

In a particular embodiment, the lipid nanocapsule according to the invention comprises:

- a solid lipid outer shell comprising the surfactants Solutol® HS15, Lipoid® S100 and optionally DSPE-PEG$_{2000}$-OCH$_3$; and
- a lipophilic liquid inner core comprising the oils Labrafac™ Lipophile WL1349 and/or Plurol® CC 497 or Peceol™, and comprising reverse micelles loaded with exenatide.

In a particular embodiment, the lipid nanocapsule according to the invention consists of:

- a solid lipid outer shell consisting of the surfactants Solutol® HS15, Lipoid® S100 and optionally DSPE-PEG$_{2000}$-OCH$_3$; and
- a lipophilic liquid inner core consisting of the oils Labrafac™ Lipophile WL1349 and/or Plurol® CC 497 or Peceol™, sodium chloride, water and reverse micelles loaded with exenatide.

In a particular embodiment, the lipid nanocapsule according to the invention comprises:

- a solid lipid outer shell comprising a poly-oxyethylene ester of fatty acid, a non-ionic lipophilic surfactant and a PEGylated lipid, in particular a PEGylated phospholipid; and
- a lipophilic liquid inner core comprising a triglyceride and a fatty acid ester, and comprising reverse micelles loaded with exenatide.

In a particular embodiment, the lipid nanocapsule according to the invention consists of:

- a solid lipid outer shell consisting of the surfactants Solutol® HS15, Lipoid® S100 and DSPE-PEG$_{2000}$-OCH$_3$; and

- a lipophilic liquid inner core consisting of the oils Labrafac™ Lipophile WL1349 and/or Plurol® CC 497 or Peceol®, sodium chloride, water and reverse micelles loaded with exenatide.

In a particular embodiment, the lipid nanocapsule according to the invention consists of:

- a solid lipid outer shell consisting of the surfactants Solutol® HS15, Lipoid® S100 and DSPE-PEG$_{2000}$-CH$_2$CH$_2$COOH; and

- a lipophilic liquid inner core consisting of the oils Labrafac™ Lipophile WL1349 and/or Plurol® CC 497 or Peceol®, sodium chloride, water and reverse micelles loaded with exenatide.

In one embodiment, the lipid nanocapsule according to the invention is formulated in two steps. In one embodiment, the method for preparing the lipid nanocapsules according to the invention comprises the steps of:

1. encapsulating one or more incretin mimetics within reverse micelles, and
2. encapsulating reverse micelles within lipid nanocapsules.

Incretin mimetic(s)-loaded reverse micelles are obtained at the end of the first step. Incretin mimetic(s)-loaded reverse micelles lipid nanocapsules are obtained at the end of the second step.

In one embodiment, the first step comprises the high-speed stirring of one or more ingredients selected in a group comprising a surfactant, an oil and a mixture thereof, preferably a surfactant and an oil (step 1a).

In one embodiment, the first step further comprises the drip of one or more incretin mimetics, preferably exenatide, into the mixture of one or more ingredients, preferably the mixture of a surfactant and an oil, described above (step 1b).

In one embodiment, the second step comprises a phase inversion process wherein ingredients of lipid nanocapsules are mixed together under magnetic stirring (step 2a). In one embodiment, ingredients of lipid nanocapsules comprise the ingredients of the solid lipid outer shell and the lipophilic liquid inner core as described herein above. In one embodiment, ingredients of lipid nanocapsules comprise oil(s), surfactant(s), salt(s) and water. In one embodiment, this step is performed at a temperature ranging from 30°C to 50°C, preferably from 35°C to 45°C, more preferably at a temperature of about 40°C. In one embodiment, this step is performed at a speed ranging from 100 rpm to 1,000 rpm, preferably at 200 rpm. In one embodiment, this step is performed for at least 1 min, 2 min or 5 min.

In one embodiment, the second step further comprises progressive heating/cooling temperature cycles (step 2b). In one embodiment, the temperature cycles were conducted at a temperature ranging from 40°C to 85°C, preferably from 45°C to 75°C, more preferably from 50°C to 68°C. In one embodiment, the reverse micelles comprising incretin mimetics obtained in step 1 are added to the mixture obtained in step 2b during the last cycle of the temperature cycles (step 2c). In one embodiment, the step 2c is performed at a temperature of about 3°C above the phase inversion zone (PIZ). In one embodiment, the step 2c is performed at a temperature ranging from about 58°C to about 70°C, preferably from about 60°C to about 68°C, more preferably from about 62°C to about 64.5°C. In one embodiment, cold water (between 0°C to 5°C, preferably around 4°C) is added to the mixture obtained at the end of step 2c after the temperature cools down reaching the PIZ in the last temperature cycle (step 2d). In one embodiment, at the end of the step 2d the temperature of the mixture is from about 57°C to about 63°C, preferably from about 58°C to about 62°C, more preferably from about 59.5°C to about 61.5°C.

In one embodiment, the method for preparing the lipid nanocapsules according to the invention does not comprise the use of any organic solvent. In other words, the method is an organic solvent-free method for preparing the lipid nanocapsules according to the invention.

In one embodiment, the lipid nanocapsules of the invention may be lyophilized and then reconstituted in the form of a colloidal suspension. In one embodiment, osmotic agents, cryoprotective agents, lyoprotective agents, preserving agents or mixtures thereof may be added to the suspension of lipid nanocapsules to be lyophilised. In one embodiment, after these compounds have completely dissolved, the suspension may undergo a first step of rapid freezing at around -50°C. In one embodiment, these suspension may then be lyophilized by direct passage of the water in the form of steam at low temperature under reduced pressure. In one embodiment, the lipid nanocapsules in dry form may then be stored in sterile form for long periods of time before use.

The invention further relates to a pharmaceutical composition comprising a therapeutically effective amount of a lipid nanocapsule as described hereinabove and a pharmaceutically acceptable vehicle.

The invention further relates to a lipid nanocapsule or a pharmaceutical composition according as described hereinabove for use as a medicament.

In one embodiment, the lipid nanocapsule according to the invention is used for the preparation of a medicament. An object of the invention is the use of a lipid nanocapsule according to the invention for the preparation of a medicament.

In one embodiment, the lipid nanocapsule, the pharmaceutical composition or the medicament according to the invention is for use in the treatment and/or the prevention of a disorder associated with a GLP-1 dysfunction.

In one embodiment, the lipid nanocapsule, the pharmaceutical composition or the medicament according to the invention is for use in the treatment and/or the prevention of a disorder selected in a group comprising type 2 diabetes mellitus (T2DM), obesity, inflammatory bowel disease (IBD), pancreatitis, dyslipidemia, non-alcoholic fatty liver disease, hyperglycemia, liver steatosis, overweight, nonalcoholic fatty liver disease (NAFLD), nonalcoholic steatohepatitis (NASH), insulin resistance, hyperinsulinemia, glucose intolerance, hyperglycemia, metabolic syndrome, prediabetes, impaired fasting glucose, hyperphagia, altered food intake behaviour, hepatic insulin resistance, whole body insulin resistance, accelerated transit, adipose tissue inflammation, cardiac dysfunctions, acute myocardial infarction, hypertension, cardiovascular disease, atherosclerosis, peripheral arterial disease, stroke, heart failure, coronary heart disease, kidney disease, diabetic complications, neuropathy, and gastroparesis.

In a particular embodiment, the lipid nanocapsule, the pharmaceutical composition or the medicament according to the invention is for use in the treatment and/or the prevention of type 2 diabetes mellitus (T2DM), obesity or inflammatory bowel disease (IBD).

In one embodiment, the lipid nanocapsule according to the invention has the advantage to significantly decrease the liver weight, the hepatic lipid accumulation and the number and size of lipid droplets.

The invention also relates to a method for treating and/or preventing a disorder associated with a GLP-1 dysfunction in a subject in need thereof comprising the oral administration of the lipid nanocapsule, the pharmaceutical composition or the medicament according to the invention to said subject.

In one embodiment, the method is for treating and/or preventing a disorder selected in a group comprising type 2 diabetes mellitus (T2DM), obesity, inflammatory bowel disease (IBD), pancreatitis, dyslipidemia, non-alcoholic fatty liver disease, hyperglycemia, liver steatosis, overweight, nonalcoholic fatty liver disease (NAFLD), nonalcoholic steatohepatitis (NASH), insulin resistance, hyperinsulinemia, glucose intolerance, hyperglycemia, metabolic syndrome, prediabetes, impaired fasting glucose, hyperphagia, altered food intake behaviour, hepatic insulin resistance, whole body insulin resistance, accelerated transit, adipose tissue inflammation, cardiac dysfunctions, acute myocardial infarction, hypertension, cardiovascular disease, atherosclerosis, peripheral arterial disease, stroke, heart failure, coronary heart disease, kidney disease, diabetic complications, neuropathy, and gastroparesis.

Another object of the invention is an *in vivo* method for increasing the blood levels of GLP-1 in an individual in need thereof comprising the oral administration to said individual of a therapeutically effective amount of a lipid nanocapsule, a pharmaceutical composition or a medicament according the invention.

Another object of the invention is an *in vivo* method for decreasing the glucose blood levels in an individual in need thereof comprising the oral administration to said individual of a therapeutically effective amount of a lipid nanocapsule, a pharmaceutical composition or a medicament according to the invention.

It will be understood that the total daily usage of the lipid nanocapsule, pharmaceutical composition and medicament of the present invention will be decided by the attending physician within the scope of sound medical judgment. The specific therapeutically effective dose level for any particular patient will depend upon a variety of factors including the disorder being treated and the severity of the disorder; activity of the specific agent employed; the specific composition employed, the age, body weight, general health, sex and diet of the patient; the time of administration, route of administration, and rate of excretion of the specific agent employed; the duration of the treatment; drugs used in combination or coincidental with the specific agent employed; and like factors well known in the medical arts. For example, it is well within the skill of the art to start doses of the agent at levels lower than those required to achieve the desired therapeutic effect and to gradually increase the dosage until the desired effect is achieved.

However, the daily dosage of the products may be varied over a wide range from about 0.01 to about 1,000 mg per adult per day, preferably 0.1 to about 500, more preferably from about 1 to about 200 mg per adult per day. In one embodiment, the lipid nanocapsule, the pharmaceutical composition or the medicament according to the invention is administered at a dosage from about 0.1 mg to about 20 mg per adult per day.

An effective amount of the lipid nanocapsule is ordinarily supplied at a dosage level from 0.1 mg/kg to about 1,000 mg/kg of body weight per day.

In one embodiment, an effective amount of the lipid nanocapsule may be administered once, twice or three times per day.

In one embodiment, the lipid nanocapsule, the pharmaceutical composition or the medicament according to the invention is administered or is adapted to be administered by oral administration.

The invention also relates to a kit for treating and/or preventing a disorder associated with a GLP-1 dysfunction comprising:

- one or more lipid nanocapsules according to the invention; and
- one or more oral hypoglycemic agent.

In one embodiment, the oral hypoglycemic agent is selected in a group comprising an α-glucosidase inhibitor, preferably acarbose, miglitol or voglibose; a biguanide, preferably metformin; a cycloset, preferably bromocriptine; a DPP-4 inhibitor, preferably sitagliptin, saxagliptin, vildagliptin, linagliptin or alogliptin; a meglitinide, preferably repaglinide or nateglinide; a SGLT2 inhibitor, preferably dapagliflozin or canagliflozin; a sulfonylurea, preferably glipizide, glyburide, gliclazide or glimepiride; a thiazolidinedione, preferably rosiglitazone or pioglitazone.

Another object of the invention is a drug delivery system for treating and/or preventing a disorder associated with a GLP-1 dysfunction comprising:

- one or more lipid nanocapsules according to the invention; and
- one or more oral hypoglycemic agent.

## BRIEF DESCRIPTION OF THE DRAWINGS

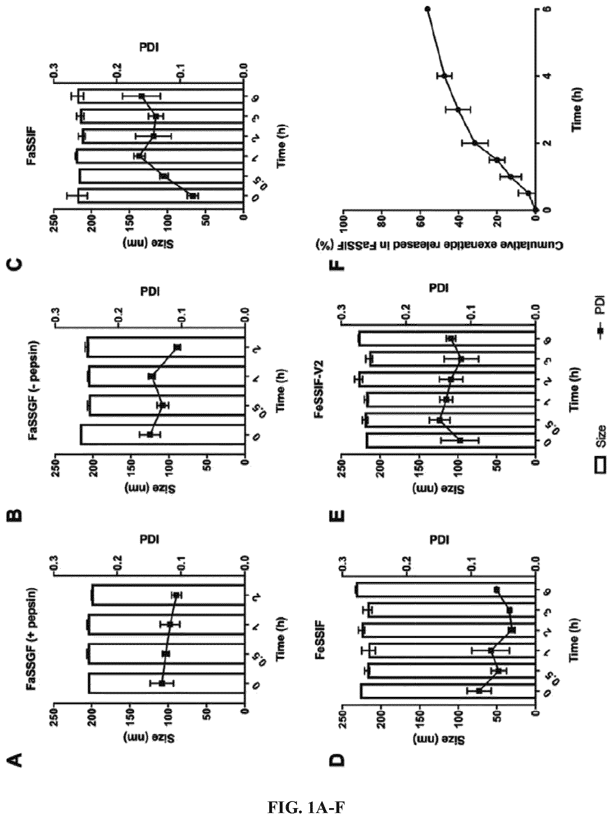

**Figures 1A-1F** are a combination of histograms and plots showing the lipid nanocapsule stability and exenatide release in biomimetic intestinal fluids. **Fig. 1A** shows the particle size and PDI of exenatide-loaded reverse micelles (RM) lipid nanocapsules (EXE RM LNC) variation following incubation in FaSSGF in the presence of pepsin at predetermined time intervals (mean ± SEM; n=9). **Fig. 1B** shows the particle size and PDI of EXE RM LNC variation following incubation in FaSSGF in the absence of pepsin at predetermined time intervals (mean ± SEM; n=9). **Fig. 1C** shows the particle size and PDI of EXE RM LNC variation following incubation in FaSSIF at predetermined time intervals (mean ± SEM; n=9). **Fig. 1D** shows the particle size and PDI of EXE RM LNC variation following incubation in FeSSIF at predetermined time intervals (mean ± SEM; n=9). **Fig. 1E** shows the particle size and PDI of EXE RM LNC variation following incubation FeSSIF-V2 at predetermined time intervals (mean ± SEM; n=9). The particle size of the EXE RM LNC was not significantly altered in any case (P>0.05) and exhibited monodispersity in the assayed media (PDI<0.2). **Fig. 1F** shows the cumulative exenatide release profile in FaSSIF (pH 6.5) at 37°C over 6 h as measured by HPLC (mean ± SEM; n=9).

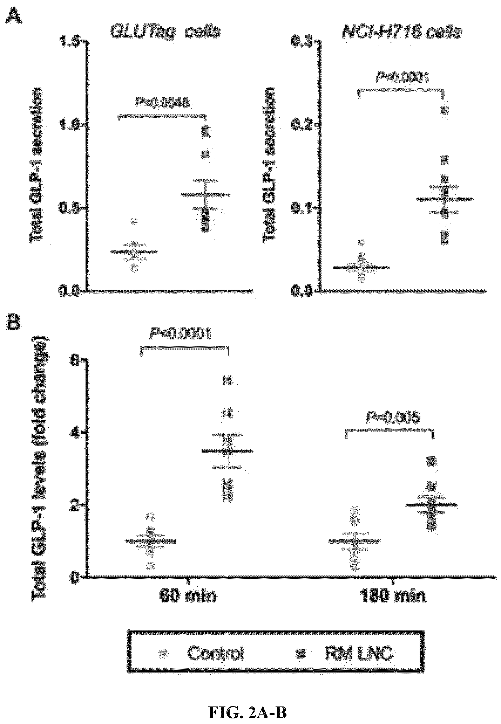

**Figures 2A-2B** are a combination of graphs showing the RM LNC-mediated GLP-1 secretion *in vitro* and *in vivo* in normoglycemic mice. **Fig. 2A** shows RM LNC-mediated *in vitro* GLP-1 secretion (2 mg/mL) in GLUTag (left) and NCI-H716 (right) cells (murine and human L cells, respectively) after a 2 h coincubation period (mean ± SEM; n=6-10). **Fig. 2B** shows *in vivo* total GLP-1 secretion in normoglycemic mice 60 and 180 min after RM LN oral administration (mean ± SEM; n=7-8). P values in **Fig. 2A** and **Fig. 2B** were determined by Student's t test or Mann-Whitney test.

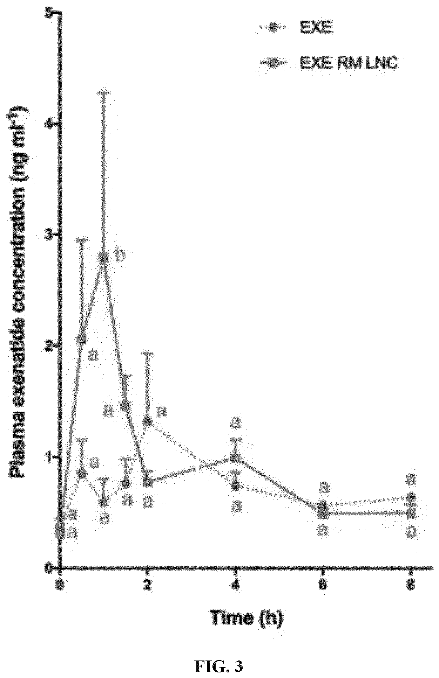

**Figure 3** represents a plot showing the exenatide plasma levels in normoglycemic mice. Exenatide blood profile is measured after oral administration to normoglycemic mice in solution in water (EXE) or encapsulation within RM LNC (EXE RM LNC) (500 µg/kg exenatide dose, corresponding to approximately 1.62 mg/g lipid nanocapsule dose) measured by ELISA (mean ± SEM; n=4). Data with different superscript letters are significantly different (P<0.05) according to two-way ANOVA followed by Tukey's post hoc test.

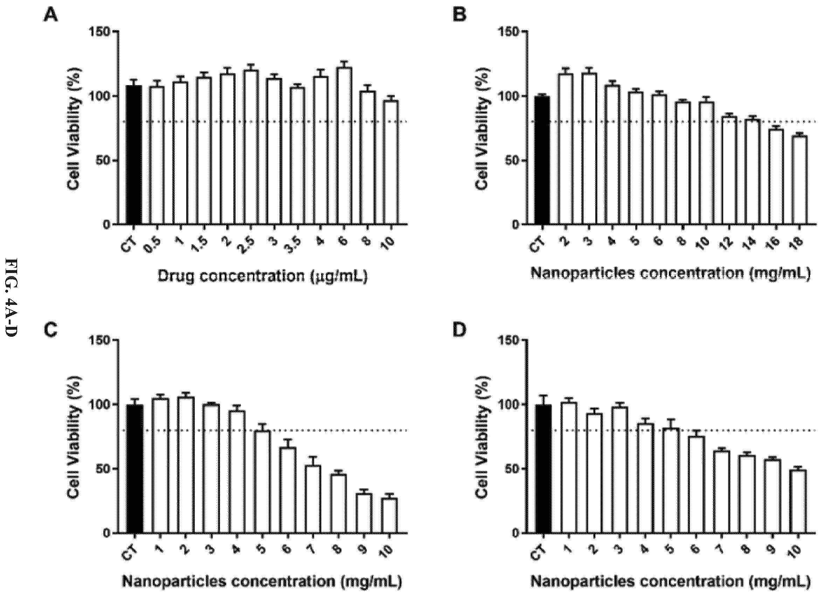

**Figures 4A-4D** are a combination of histograms showing the *in vitro* cytotoxicity studies in L cells and enterocyte-like cells. **Fig. 4A** shows the cell viability of EXE RM LNC on Caco-2 cells after incubation for 2 h at 37°C expressed as cell viability with respect to drug concentration. **Fig. 4B** shows the cell viability of EXE RM LNC on Caco-2 cells after incubation for 2 h at 37°C expressed as cell viability with respect to nanoparticle concentration. **Fig. 4C** shows the cell viability of RM LNC on GLUTag cells after coincubation with increasing lipid nanocapsule concentrations (1 mg/mL to 10 mg/ml) for 2 h. **Fig. 4D** shows the cell viability of RM LNC on human NCI-H716 cells after coincubation with increasing lipid nanocapsule concentrations (1 mg/mL to 10 mg/ml) for 2 h. Data are shown as the mean ± SEM (n=9). The dotted line corresponds to a viability of 80%. The data correspond to three independent experiments.

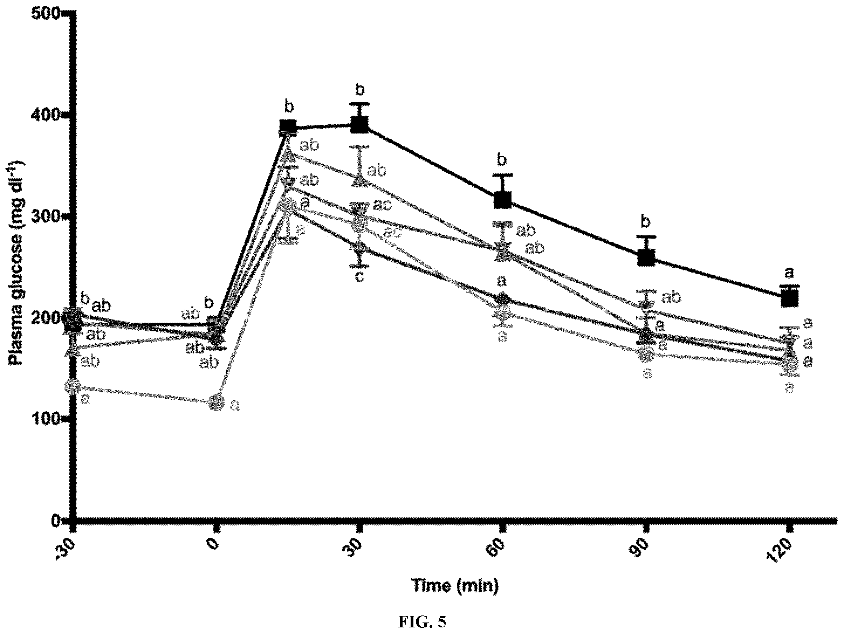

**Figure 5** represents plots showing the plasma glucose levels (mg/dl) measured 30 min before and 120 after glucose challenge (n= 8-9). Data with different superscript letters are significantly different (P<0.05) according to a two-way ANOVA followed by Tukey's post hoc test.

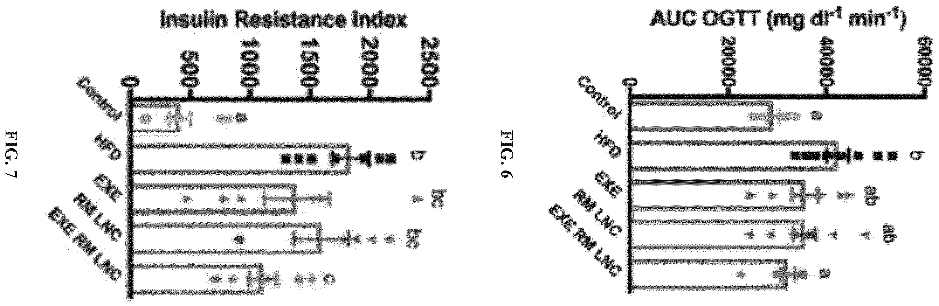

**Figure 6** represents histograms showing the mean area under the curve (AUC, mg/dl/min) measured 30 min before and 120 after glucose challenge (n= 8-9).

**Figure 7** represents histograms showing the insulin resistance index determined by multiplying blood glucose AUC by insulin AUC (n= 8-9).

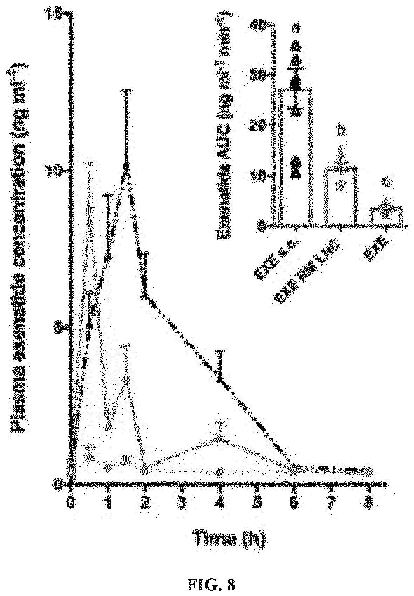

**Figure 8** represents plots showing the concentration-time profile and AUC of exenatide after subcutaneous administration (EXE s.c.) (50 µg/kg exenatide dose) and oral administration, in solution and within RM LNC (EXE and EXE RM LNC, respectively) (500 µg/kg exenatide dose corresponding to approximately 1.62 mg/g lipid nanocapsule dose). Data are presented as the mean ± SEM (n=8-10). Data with different superscript letters are significantly different (P<0.05) according to a one-way ANOVA followed by Tukey's post hoc test.

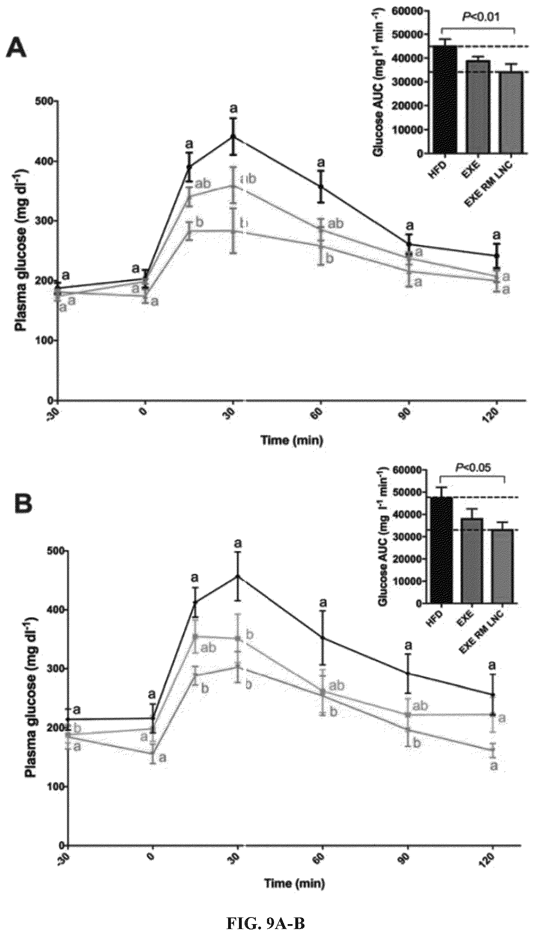

**Figures 9A-9B** are a combination of plots showing the OGTT evaluation of EXE RM LNC in 8- and 10-week HFD-induced diabetic mice. **Fig. 9A** shows plasma glucose levels and AUC following a 2 g/kg glucose oral challenge measured in mice (C57BL6/J mice) fed a control diet and a HFD (8 weeks). **Fig. 9B** shows plasma glucose levels and AUC following a 2 g/kg glucose oral challenge measured in mice (C57BL6/J mice) fed with control diet and with HFD (10 weeks) determined by a two-way ANOVA. Data are presented as the mean $\pm$ SEM (n= 7-8). Values with different superscript letters are significantly different (P<0.05). P values were determined by two-way ANOVA for the OGTT and by one-way ANOVA followed by Tukey's post hoc test for comparing the AUC between groups.

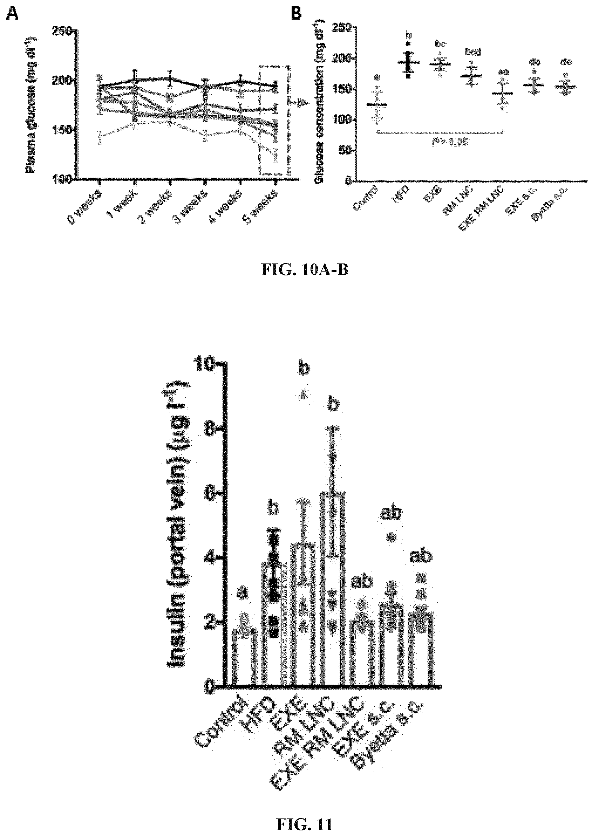

**Figures 10A-10B** are a combination of plots showing the effect of EXE RM LNC on glucose homeostasis in obese/diabetic mice. **Fig. 10A** shows plasma glucose levels over a 5 weeks treatment (13 weeks of HFD feeding), expressed in mg/dl. **Fig. 10B** shows plasma glucose concentration after 5 weeks of treatment (13 weeks of HFD feeding), expressed in mg/dl. Data are presented as the mean $\pm$ SEM (n=10). Data with different superscript letters are significantly different (P<0.05). P values were determined by a two-way ANOVA followed by Tukey's post hoc test.

**Figure 11** represents histograms showing the effect of EXE RM LNC on hyperinsulinemia in obese/diabetic mice. Insulin plasma levels were measured from the portal vein (n=8-10). Data are presented as the mean $\pm$ SEM. Data with different superscript letters are significantly different (P<0.05). P values were determined by Kruskal-Wallis followed by Dunn's post hoc test.

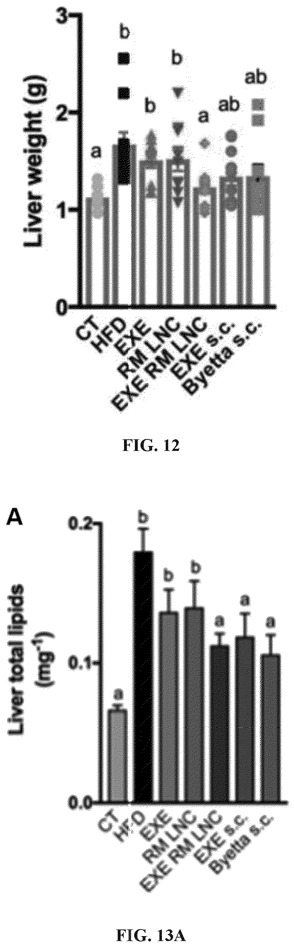

**Figure 12** represents histograms showing the impact of EXE RM LNC treatment on liver weight (g). Data with different superscript letters are significantly different (P<0.05). P values were determined following one-way ANOVA with Tukey's post hoc test.

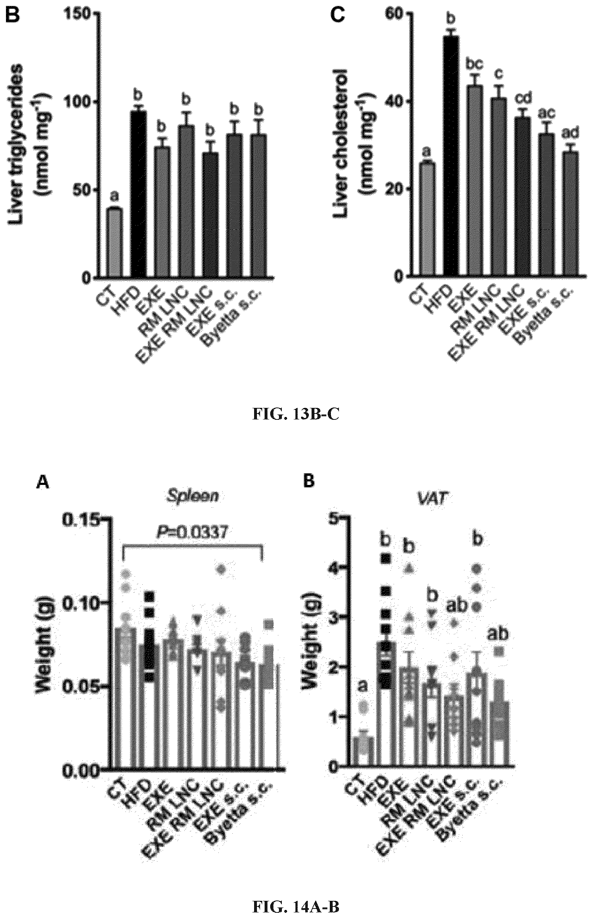

**Figures 13A-13C** are a combination of histograms showing the impact of EXE RM LNC treatment on lipid homeostasis. **Fig. 13A** shows liver total lipid content (mg$^{-1}$ per 100 mg of tissue). **Fig. 13B** shows liver triglycerides (nmol.mg$^{-1}$). **Fig. 13C** shows liver cholesterol (nmol.mg$^{-1}$). Data with different superscript letters are significantly different (P<0.05). P values were determined by the Kruskal-Wallis test followed by Dunn's post hoc test.

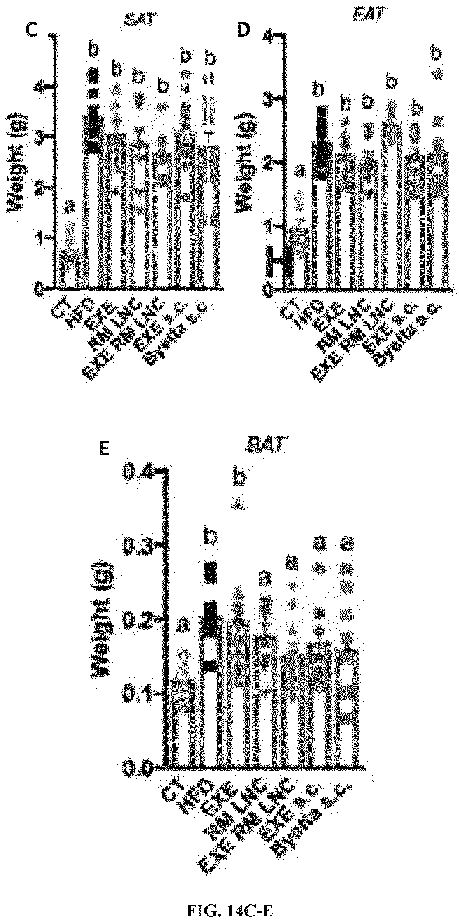

**Figures 14A-14E** are a combination of histograms. **Fig. 14A** shows the impact of EXE RM LNC treatment on the weights of the spleen. **Fig. 14B** shows the impact of EXE RM LNC treatment on the weights of visceral adipose tissue (VAT). **Fig. 14C** shows the impact of EXE RM LNC treatment on the weights of subcutaneous adipose tissue (SAT). **Fig. 14D** shows the impact of EXE RM LNC treatment on the weights of epicardial adipose tissue (EAT). **Fig. 14E** shows the impact of EXE RM LNC treatment on the weights of brown adipose tissue (BAT). Data are presented as the mean $\pm$ SEM (n=9-10). Data with different superscript letters are significantly different (P<0.05). P values were determined following one-way ANOVA with Tukey's post hoc test.

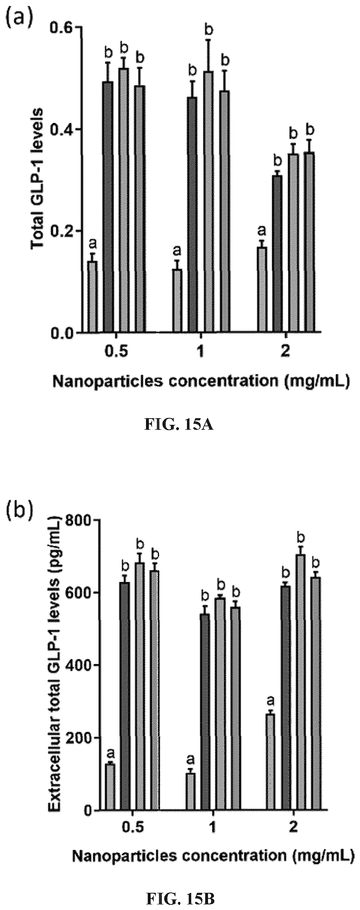

**Figures 15A-15B** are a combination of histograms, showing the effect of PEGylated nanocapsules (with or without propionate grafted as a ligand) on GLP-1 stimulation in GLUTag cells (murine L cells) and in normoglycemic mice. Effect of PEGylated RM LNC (with or without propionate grafted as a ligand) on GLP-1 stimulation in murine GLUTag cells after 2 h of co-incubation with increasing nanocapsule concentrations from 0.5 to 2 mg/mL (mean $\pm$ SEM; n=4; N=3). Are depicted for each panels and for each concentration assayed, from left to right: medium, RM LNC, RM LNC PEG and RM LNC PEG-PRO. **Fig. 15A** (a) represents the total GLP-1 levels; **Fig. 15B** (b) represents the extracellular total GLP-1 levels (expressed in pg/mL).

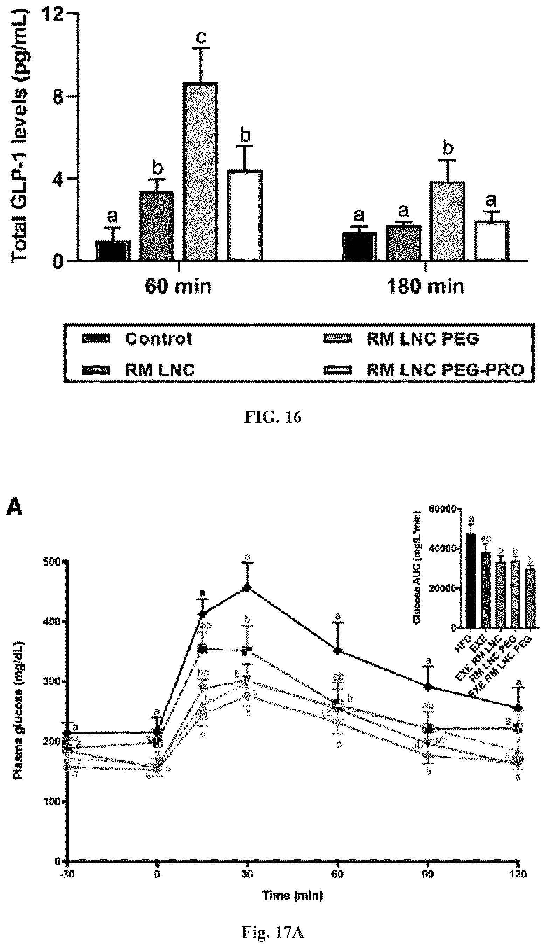

**Figure 16** is a histogram showing the total GLP-1 levels in healthy control mice 60 and 180 min post-oral gavage with medium as controle (black bars) RM LNC (dark grey bars), RM LNC PEG (light grey bars) and RM LNC PEG-PRO (white bars) using the same nanocapsule dose (1.62 mg/g) (mean $\pm$ SEM; n=8). Different superscript letters represent significant differences between the groups (*p<0.05) based on one-way ANOVA followed by Tukey's post hoc test.

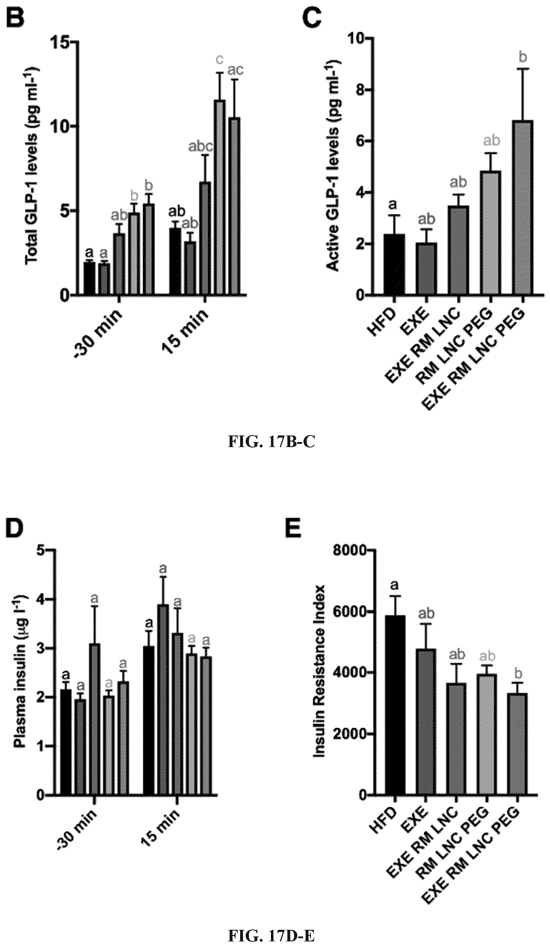

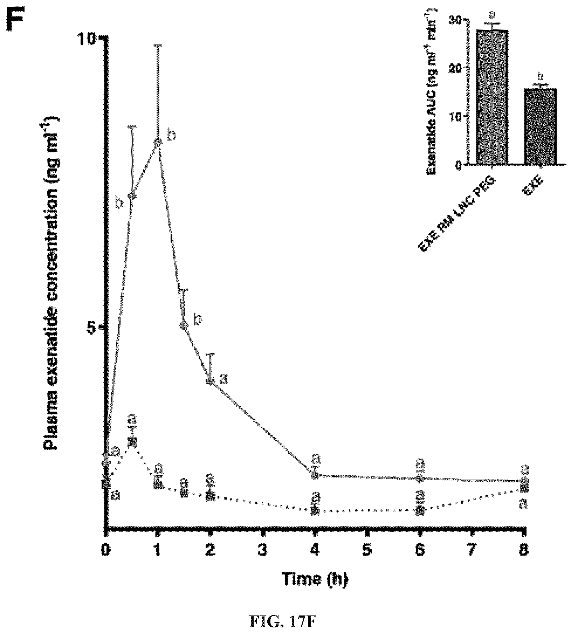

**Figure 17A-17F** is a combination of graphs and histograms showing the pharmacological and pharmacokinetics studies in obese/diabetic mice after a single orally administered dose. **Fig. 17A:** Blood glucose values (mg/dL) and mean AUC (mg/dL·min) were tested 30 min before and 120 min after glucose administration (n=7-8). HFD (black diamonds); EXE (squared); EXE RM LNC (inverted triangles); RM LNC PEG (triangles); EXE RM LNC PEG (grey diamonds). **Fig. 17B:** Plasma total GLP-1 concentrations were measured 30 min before and 15 min after glucose administration (n=6-8). For the order of the conditions, see insert of **Fig. 17A. Fig. 17C:** Active GLP-1 levels measured in the portal vein of obese/diabetic mouse after OGTT (3 h post-administration of the formulation) (n=6-8). **Fig. 17D:** Insulin concentrations were measured in plasma collected from blood from the tail vein 30 min before and 15 min after oral glucose administration (n=6-8). For the order of the conditions, see insert of of **Fig. 17A** or **Fig. 17C. Fig. 17E:** Insulin resistance index (n=7-8). **Fig. 17F:** Plasma exenatide concentration-time profile and exenatide AUC in diabetic mice (10 weeks of HFD feeding) after oral administration of the drug solution or drug-loaded PEGylated nanocapsules (EXE and EXE RM LNC PEG, respectively) (dose: 500 $\mu$g/kg) (n=9-10). Data are shown as the mean $\pm$

SEM. Different superscript letters indicate a significant difference among the groups (*p<0.05) by two-way analysis of variance (ANOVA) and Tukey's post hoc test **(Fig. 17A, F),** the Kruskal-Wallis followed by Dunn's post hoc test **(Fig. 17B)** or one-way ANOVA followed by Tukey's post hoc test **(Fig. 17C-E).**

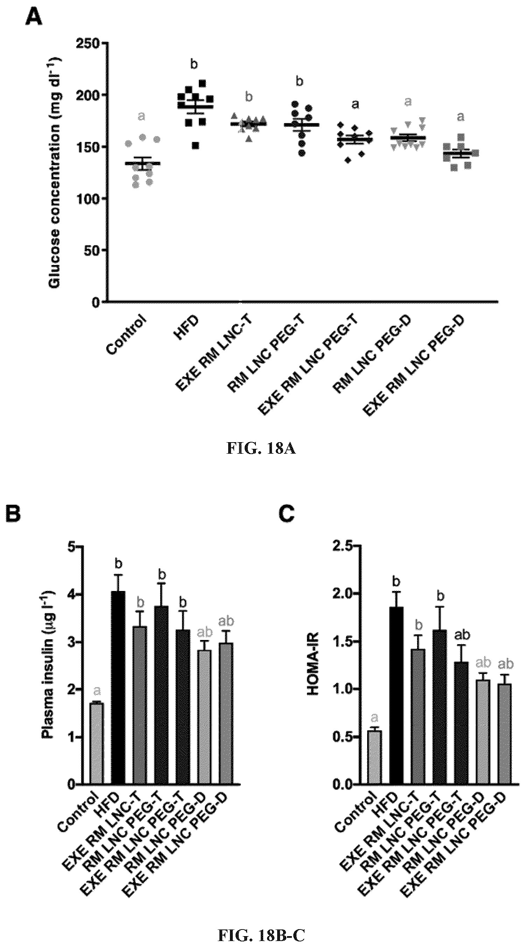

**Figure 18A-18C** is a combination of graphs showing the effect of PEGylated and non-PEGylated EXE-loaded lipid nanocapsules with different oral administration frequencies on the suppression of hyperglycemia and hyperinsulinemia in HFD diet-induced diabetic mice via long-term therapy. **Fig. 18A:** Plasma glucose values (mg/dL) after 4 weeks of administration (HFD feeding for 14 weeks in total) (mean $\pm$ SEM; n=7-10). **Fig. 18B:** Insulin concentrations were tested in the plasma from blood retrieved from the tail vein (mean $\pm$ SEM; n=6-9). **Fig. 18C:** HOMA-IR was calculated using the equation [fasting glucose in mg/dL $\times$ fasting insulin in ng/mL]/405 as previously defined in Amrutkar et al. (Diabetes. 2015; 64:2791-2804) (mean $\pm$ SEM; n=8-9). Different superscript letters show statistically significant differences among the groups (*p<0.05) based on two-way analysis of variance followed by Tukey's post hoc test **(Fig. 18A),** or the Kruskal-Wallis followed by Dunn's post hoc test **(Fig. 18B-C).**

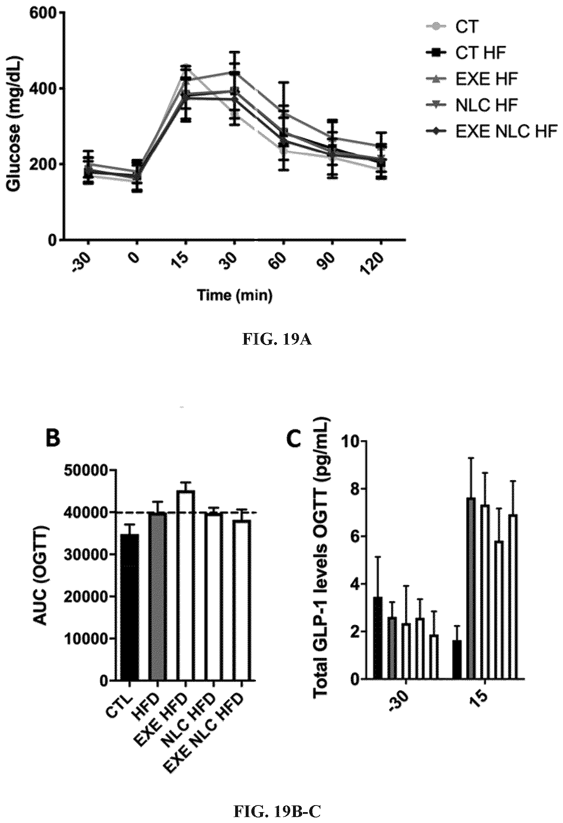

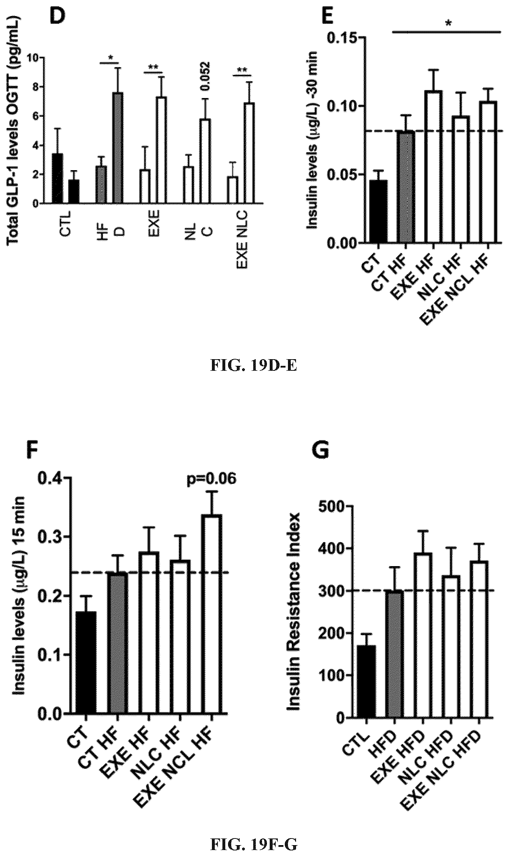

**Figure 19A-19G** is a combination of graphs showing the plasma glucose levels (mg/dL) measured 30 min before and 120 after glucose challenge (n= 9-10) **(Fig. 19A)** and mean area under the curve (AUC, mg/dL/min) measured 30 min before and 120 after glucose challenge (n= 9-10) **(Fig. 19B)** via nanostructured lipid carriers (NLC). Plasma total GLP-1 levels **(Fig. 19C-D)** and plasma insulin levels **(Fig. 19E-F)** measured 30 min before and 15 min after glucose challenge. **(Fig. 19G)** Insulin resistance index (n= 8-9). Data are presented as the mean $\pm$ SEM (n=8-10).

## EXAMPLES

The present invention is further illustrated by the following examples.

## Example 1: Working example 1

### 1. Materials and Methods

#### 1.1 - *Study design*

The aim of this study was to develop a nanocarrier-based drug delivery system for the oral delivery of incretin mimetic peptides. The enhancement of endogenous GLP-1 secretion represents a more physiological and novel alternative in incretion-based diabetes therapy. It was hypothesized that increasing endogenous GLP-1 secretion alone would not be sufficient to induce a therapeutic effect in the pathological context. Hence, focus was placed on engineering a dual-action drug delivery nanosystem that synergizes its own biological effect (inducing endogenous GLP-1 secretion) and the effect of the encapsulated incretin mimetic peptide (increasing peptide bioavailability) as an alternative oral treatment for T2DM. The physicochemical properties of this novel nanosystem was characterized (size, zeta potential) and the encapsulation efficiency, stability and release of the encapsulated peptide (exenatide) in biomimetic media *in vitro* were confirmed. The ability of the nanosystem per se to induce GLP-1 secretion was evaluated *in vitro* in murine and human cell lines and *in vivo* in normoglycemic mice. In addition, a pharmacokinetic study was conducted in normoglycemic mice to confirm enhanced bioavailability when encapsulated within the nanosystem as compared to the peptide alone. Once the proof of concept regarding the efficacy of the nanocarrier at inducing GLP-1 was obtained, the utility of the dual-action nanosystem on reducing hyperglycemia was evaluated in diabetic and obese mice following an acute treatment (one single dose) after 3, 8 or 10 weeks of HFD feeding, and the increased bioavailability of the encapsulated peptide within the nanosystem was confirmed in obese/diabetic mice. Finally, the impact of the nanosystem on glucose homeostasis and lipid metabolism was studied in an obese/diabetic mouse model following chronic long-term treatment (once daily for 5 weeks) to confirm that this nanosystem represents a viable alternative to current strategies for the oral delivery of incretin peptides in the treatment of T2DM. For treatment and pharmacokinetic studies in obese/diabetic mice, 10 mice were used per group on the basis of previous studies. All animal experiments were approved by and performed in accordance with the local animal committee (2014/UCL/MD/033 and 2017/UCL/MD/005) and as specified by the Belgian Law of 29 May 2013 on the protection of laboratory animals.

#### 1.2 - *Materials*

Exenatide was purchased from Bachem (Bubendorf, Switzerland). Labrafac® WL 1349 (caprylic/capric acid triglycerides) and Peceol® (oleic acid mono-, di- and triglycerides) were from Gattefossé (Saint-Priest, France). Lipoid® S 100 (soybean lecithin at 94% of phosphatidylcholines) was from Lipoid GmbH (Ludwigshafen, Germany). Solutol® HS15 (mixture of free PEG 660 and PEG 660 12-hydroxystearate, Mw 870 Da) and Span® 80 (sorbitan oleate) were purchased from Sigma-Aldrich (St. Louis, USA). Sodium chloride (NaCl), lecithin, sodium taurocholate, pepsin, 3-(4,5- dimethyl-thiazol-2-yl)-2,5-diphenyl tetrazolium bromide (MTT), dimethyl sulfoxide (DMSO) and Triton-X 100 were purchased from Sigma-Aldrich (St. Louis, USA). Total GLP-1 (ver.2) and active GLP-1 assay kits were purchased from Meso Scale Discovery (Maryland, USA). An Exendin-4 enzyme immunoassay kit was purchased from Phoenix Europe GmbH (Karlsruhe, Germany). An Ultrasensitive Mouse Insulin ELISA Kit was purchased from Mercodia AB (Uppsala, Sweden). Matrigel® was obtained from BD Bioscience (Belgium). Dipeptidyl peptidase IV (DPP-IV) inhibitor was purchased from Millipore (St. Charles, USA). Dulbecco's modified Eagle's medium (DMEM)-GlutaMAX (5.5 Mm glucose), Roswell Park Memorial Institute (RPMI)-1640 medium, penicillin-streptomycin (P/S), fetal bovine serum (FBS), phosphate buffered saline (PBS), trypsin (0.25%) containing ethylenediaminetetraacetic acid (EDTA, 0.02%) were also used and purchased from Thermo Fisher Scientific (Invitrogen, Belgium). All chemical regents utilized in this study were of analytical grade.

### 1.3 - Preparation and characterization of reverse micelle-loaded lipid nanocapsules

Reverse micelle-loaded lipid nanocapsules (RM LNC) were formulated in two steps, in which the drug was first encapsulated within reverse micelles and then further encapsulated within LNC. First, exenatide-loaded reverse micelles (EXE RM) were prepared by high-speed stirring of a surfactant (Span® 80) and an oil (Labrafac® WL 1349) mixture (1:5 weight ratio). Then, 50 µL of EXE (30 mg/ml in MilliQ water) was dripped into the mixture and maintained under stirring. Exenatide-loaded reverse micelle lipid nanocapsules (EXE RM LNC) were prepared following a modified phase inversion process described by Heurtault et al. (Pharm Res. 2002; 19:875-880). Briefly, all components (shown in **Table 1),** including lipophilic Labrafac® WL 1349, Peceol®, Lipoid® S100, Solutol® HS15, sodium chloride (NaCl) and MilliQ water, were mixed together under magnetic stirring at 40°C at 200 rpm for 5 min.

Table 1: Composition of EXE RM LNC

| Composition | Reverse micelles (RM) | Lipid nanocapsules (LNC) |
| --- | --- | --- |
| Drug solution (µL) | 50 | - |
| Span® 80 (mg) | 100 | - |
| Labrafac® WL 1349 (mg) | 500 | 769.5 |
| Peceol® (mg) | - | 85.5 |
| Lipoid® S100 (mg) | - | 13.4 |
| Solutol® HS15 (mg) | - | 120 |
| Sodium chloride (mg) | - | 50 |
| MilliQ water (µl) | - | 1025 |
| Cold milliQ water (µl) | - | 2500 |

Temperature cycles of progressive heating/cooling were conducted between 50°C and 67°C. During the last cycle, 500 µL of preheated drug loaded RM was added to the mixture at approximately 3°C above the phase inversion zone (PIZ; 59°C to 61.5°C). The solution was cooled to reach the phase inversion zone (PIZ) temperature, and 2.5 ml of cold MilliQ water (4°C) was added and stirred at high speed for 2 min. Blank RM LNC were prepared following the same protocol in the absence of exenatide.

### 1.4 - Quantification of exenatide

The exenatide encapsulated within RM LNC was quantified by high-performance liquid chromatography (HPLC, Shimadzu, Japan) using a gradient method as previously described by Shrestha *et al.* (Nanoscale. 2018; 10:603-613). Briefly, a Kinetex® EVO C18 column (100 Å, 2.6 µm, 150 x 4.6 mm) (Phenomenex, USA), with a security guard column (Phenomenex, USA) was used at room temperature. The aqueous mobile phase comprised 0.05% (v/v) trifluoroacetic acid (TFA) in water, and the organic mobile phase consisted of 0.05% (v/v) in acetonitrile. A gradient system was developed with an initial ratio of 10:90 (v/v, aqueous:organic phase) at a flow rate of 1 mL/min, which was linearly changed to 90:10 (v/v) over 10 min and kept constant for the next minute. Then, the ratio was linearly changed to the initial composition in the next 1.5 min and was stabilized for the last minute. The injection volume used was 20 µL, and the detection wavelength used was 220 nm. The retention time was 5.9 min, and the limit of detection and limit of quantification were $1.1 \pm 0.4$ µg/mL and $3.3 \pm 1.1$ µg/mL, respectively.

*1.5 - Characterization of EXE RM LNC*

EXE RM LNC were characterized by measuring their particle size and polydispersity index (PDI) by dynamic light scattering (DLS) using a Zetasizer Nano ZS (Malvern Instruments Ltd., Worcestershire, UK). The zeta potential was determined by laser Doppler velocimetry (LDV) using a Zetasizer Nano ZS. For the measurement, 5 $\mu$L of lipid nanocapsule suspension was dispersed in 995 $\mu$L of ultrapure water. All measurements were performed in triplicate.

EXE RM LNC were also characterized based on their drug encapsulation efficiency (EE, %). To calculate the total drug content, 50 $\mu$L of EXE RM LNC were dissolved in 950 $\mu$L of methanol followed by strong vortexing. Free and encapsulated exenatide were separated by ultrafiltration using Amicon® centrifuge filters (MWCO 30 kDa, 4000g, 4°C, 20 min) (Millipore). Filtrates were further diluted using a 1:2 dilution factor. The exenatide in the filtrate and the exenatide dissolved in methanol were quantified using the above-described HPLC method. The EE was calculated using the following equation:

$$EE\ (\%) = (\text{total amount of exenatide} - \text{free exenatide}) / (\text{total amount of exenatide}) \times 100.$$

*1.6 - Lipid nanocapsule stability and drug release in stimulated gastrointestinal fluids*

The *in vitro* stability of EXE LNC was tested in five different biomimetic media: Fasted State-Simulated Gastric Fluid (FaSSGF) with and without pepsin, Fasted State-Simulated Intestinal Fluid (FaSSIF), Fed State-Simulated Intestinal Fluid (FeSSIF) and FeSSIF version 2 (FeSSIF-V2) (biorelevant.com, UK). A detailed description of the composition of the simulated fluids used is presented in **Table 2.**

**Table 2:** Composition of biomimetic intestinal fluids

| Composition | FaSSGF | FaSSIF | FeSSIF | FeSSIF-V2 |
|---|---|---|---|---|
| Sodium taurocholate | 0.08 mM | 3 mM | 15 mM | 10 mM |
| Lecithin | 0.02 mM | 0.75 mM | 3.75 mM | 2 mM |
| Glycerol monooleate | - | - | - | 5 mM |
| Sodium oleate | - | - | - | 0.8 mM |
| Sodium chloride | 34.2 mM | 34.2 mM | 203.18 mM | 126 mM |
| Sodium monobasic phosphate anhydrous | - | 28.65 mM | - | - |
| Sodium hydroxide | - | 8.71 mM | 101.02 mM | 82 mM |
| Acetic acid | - | - | 144.04 mM | - |
| Maleic acid | - | - | - | 55 mM |
| Pepsin | 0.1 mg/mL | - | - | - |
| pH | 1.6 | 6.5 | 5 | 5.8 |

The influence of gastric and intestinal conditions on lipid nanocapsule stability was evaluated based on the lipid nanocapsule size and the PDI. EXE RM LNC were incubated in FaSSGF with and without pepsin, FaSSIF, FeSSIF and FeSSIF-V2 at 37°C (100 $\mu$L of lipid nanocapsules in 10 mL of media) under gentle stirring. At predetermined time intervals (0, 0.5, 1 and 2 h for stimulated gastric media and 0, 0.5, 1, 3 and 6 h for stimulated intestinal media and FeSSIF), samples were withdrawn and then analyzed by DLS.

*1.7 - In vitro drug release studies*

The drug release from EXE RM LNC was evaluated in FaSSGF in the absence of pepsin and in FaSSIF media for 2 h and 6 h, respectively. Studies were performed using the dialysis method. Briefly, 1 mL of EXE RM LNC was placed in disposable dialysis membranes (MWCO 100 kDa) (Float-A-Lyzer®G2, Microfloat, Spectrum labs, USA) and introduced into 50 ml falcon tubes containing 35 ml of medium at 37 °C under magnetic stirring. At predetermined times, 50 $\mu$L of sample was withdrawn and dissolved in 950 $\mu$L of methanol. The concentration of exenatide was determined by the HPLC, as described above.

*1.8 - In vitro cell studies*

*1.8.1 - Cell cultures*

The human NCI-H716 L cell line was obtained from the American Type Culture Collection (ATCC) (Manassas, VA) and was used from passages 15 to 20. The completed media was composed of Roswell Park Memorial Institute (RPMI) 1640 medium with 1% (v/v) penicillin-streptomycin (P/S) and 10% (v/v) fetal bovine serum (FBS). Cells were suspended growth in 75 cm$^2$ flasks (Corning, Lowell, MA, USA) in an atmosphere of 5% $CO_2$/95% air (v/v) at 37°C. Some fresh medium was added every other day. After replacement of the medium, cultured were centrifuged and subsequently resuspended at a proper density.

Intestinal murine L cell line GLUTag cells were used from passages 16 to 29. Cells were grown in DMEM GlutaMAX (5.5 mM glucose) supplemented with 10% (v/v) inactivated FBS and 1% (v/v) P/S (complete DMEM medium) at 37°C with 5% $CO_2$ supply. Cells were subcultured using trypsin (0.25%) containing EDTA (0.02%) every 4-5 days.

Caco-2 cells (clone-1) were used from passages 25 to 30. The Caco-2 cell line was maintained in medium consisting of DMEM supplemented with 10% (v/v), HyCloneTM FBS, 1% (v/v) L-Glutamine, 1% (v/v) nonessential amino acids and 1% (v/v) P/S at 37°C in an atmosphere of 10% $CO_2$/95% air (v/v). The medium was replaced every other day.

*1.8.2 - Cytotoxicity studies*

*In vitro* cytotoxicity studies of EXE RM LNC were performed in Caco-2 cells based on the drug and lipid nanocapsule concentration calculated as previously described using 3-(4,5-dimethylthiazol-2-yl)-(2,5-diphenyltetrazolium bromide) (MTT) colorimetric assay (Beloqui et al., Journal of Controlled Release. 2013; 166, 115-123). The influence of unloaded RM LNC on cell viability was also tested in GLUTag and NCI-H716 cells. Caco-2 cells ($5 \times 10^4$ cells per well) were seeded in 96-well tissue culture plates (Costar Corning CellBIND Surface, USA) and were allowed to adhere overnight. For cytotoxicity studies in GLUTag and NCI-H716, $5 \times 10^4$ cells per well were seeded on Matrigel®-coated (10 μL/mL of medium) 96-well plates. After washing the plates with prewarmed PBS buffer (x3), 100 μL of EXE RM LNC at increasing drug concentrations (0.5-10 mg/mL), corresponding to increasing lipid nanocapsule concentrations (2-18 mg/mL), were dispersed in DMEM (without FBS) and co-incubated with Caco-2 cells for 2 h at 37°C. Increasing unloaded RM LNC concentrations (from 1 mg/ml to 10 mg/ml) were dispersed in DMEM GlutaMAX or RPMI-1640 medium (without FBS) and co-incubated with GLUTag or NCI-H716 cells, respectively, at 37°C for 2 h. After incubation, the supernatants were replaced by 100 μL of 0.5 mg/ml MTT for 3 h. The purple formazan crystals were dissolved in 200 μL of DMSO for absorbance determination at 560 nm using a MultiSksan EX plate reader (Thermo Fisher Scientific, USA). Cells with Triton-X 100 (100% dead) and cells with culture medium (100% alive) were considered positive and negative controls, respectively. Tests were performed in triplicate.

*1.9 - In vitro GLP-1 secretion*

GLUTag and NCI-H716 cells ($1.8 \times 10^5$ cells per well) were seeded onto Matrigel®-coated 24-well cell culture plates and allowed to adhere for 24 h. The day after, the plate was gently washed using prewarmed PBS. Then, the GLUTag cells were co-incubated with DMEM GlutaMAX without FBS or unloaded RM LNC. On the other hand, NCI-H716 cells were incubated with RPMI-1640 medium without FBS and unloaded RM LNC. Both media contained DPP-IV inhibitor at a final concentration of 50 μM (Millipore, St. Charles, MO, USA). To confirm the efficacy of the lipid nanocapsules compared with previously tested lipid nanocapsules (Xu et al., Mol Pharm. 2018; 15, 108-115), we used a 2 mg/ml lipid nanocapsule concentration. After 2 h of incubation at 37°C, supernatants were collected, centrifuged at 250 g for 5 min at 4°C (Centrifuge 5804 R, Eppendorf AG, Hamburg, Germany) and preserved at -80°C until further analysis. Cells were collected in PBS in the presence of DPP-IV inhibitor. Cell extracts containing GLP-1 were obtained after three freeze-thaw cycles followed by centrifugation at 250 g for 5 min at 4°C. Total GLP-1 concentration was determined using a Total GLP-1 ELISA kit (Meso Scale Delivery, Gaithersburg, USA). GLP-1 secretion is expressed as the amount of GLP-1 detected in the supernatants plus cells. GLP-1 secretion was calculated by the following equation:

$$\text{GLP-1 secretion} = C_{\text{extracellular}} / (C_{\text{intracellular}} + C_{\text{extracellular}})$$

where $C_{\text{extracellular}}$ is the concentration of GLP-1 tested in the supernatants, and $C_{\text{intracellular}}$ is the concentration of GLP-1 tested in cells.

*1.10 - Total GLP-1 secretion in normoglycemic mice*

Normoglycemic mice (C57BL/6J male mice, 20-25 g, 10 weeks; Janvier Laboratories, France) were randomly divided into two groups with 8 mice each. Animals were fasted overnight before the experiments with free access to water. Mice were treated with blank RM LNC corresponding to approximately 1.62 mg/g nanoparticle dose. Control mice were treated by oral gavage with an equivalent volume of MilliQ water. Blood samples were withdrawn from the tip of the tail vein at 60 and 180 min after oral administration. Samples were collected in the presence of DPP-IV inhibitor (20 μL per ml of blood) and maintained on ice. Immediately after the study, blood samples were centrifuged (3,000 rpm, 10 min at 4°C), and the plasma was kept frozen at -80°C until analysis. The total GLP-1 levels were quantified using a Total GLP-1 ELISA kit. (Meso Scale Delivery, USA). The total GLP-1 plasma values are expressed as the fold-change compared with the untreated control group.

### 1.11 - *Oral glucose tolerance test in high-fat diet induced obese/diabetic mice*

Eight-week-old male mice were housed 5 per cage and divided into 5 groups (10 mice per group). After 2 weeks of acclimation, mice were fed a high-fat diet) (60% fat and 20% carbohydrates (kcal per 100 g), D12492i, Research Diets, USA) (HFD and exenatide-treated groups) or a normal chow diet (control, AIN93Mi, Research Diets, USA) for 3, 8 or 10 weeks before experiments and underwent a period of fasting overnight before being treated with an oral exenatide in solution (EXE, 500 μg of exenatide/kg body weight), exenatide-reverse micelle-loaded lipid nanocapsules (EXE RM LNC, 500 μg of exenatide/kg body weight) or unloaded micelle-loaded lipid nanocapsules (RM LNC, equivalent concentrations as per EXE RM LNC) 1 h before being challenged with an oral glucose gavage. Control groups (control diet and HFD groups) were treated with an oral gavage of an equivalent volume of sterile MilliQ water. After 1 h, mice were challenged with an oral glucose gavage (2 g/kg glucose dose). Blood glucose was measured 30 min before oral glucose (-30 min) and at 0, 15, 30, 90 and 120 min after oral glucose load. Blood glucose was determined with a glucose meter (Accu Check, Roche, Switzerland) from blood samples collected from the tip of the tail vein. Blood samples were collected at -30 min and 15 min to test total GLP-1 and insulin plasma concentration by using ELISA kits (Meso Scale Delivery, USA and Mercodia, Uppsala, Sweden, respectively). The insulin resistance index was determined by multiplying the area under the curve of both blood glucose and insulin in the plasma obtained by the oral glucose tolerance test (OGTT).

### 1.12 - *Pharmacokinetics study in normoglycemic and obese/diabetic mice*

Eight-week-old male mice were randomly divided into three groups (10 mice per time point) and housed in a controlled environment (room temperature of $23 \pm 2°C$, 12 h daylight cycle) with free access to food and sterile water. After two weeks of acclimation, mice underwent 3 weeks of HFD (60% fat). Prior to the experiments, mice were fasted overnight with free access to sterile Milli-Q water. Exenatide in solution and EXE RM LNC were orally administered at a 500 μg/kg dose. Exenatide was also subcutaneously administered at a 50 μg/kg dose. At different time points (0, 0.5, 1, 1.5, 2, 4, 6 and 8 h), blood samples were collected from the tip of the tail vein. Blood samples were then centrifuged (1,500 g, 10 min at 4°C), and exenatide plasma concentration was quantified using an ELISA kit (EK-070-94, Phoenix Europe GmbH, Karlsruhe, Germany). The relative bioavailability (FR %) of exenatide was calculated using the following equation:

$$FR(\%) = \frac{AUC_{oral} \times Dose_{s.c.}}{AUC_{s.c.} \times Dose_{oral}} \times 100$$

The pharmacokinetic parameters were analyzed using PKSolver (Zhang et al., Comput Methods Programs Biomed. 2010; 99, 306-314). In the case of normoglycemic mice, 4 mice per time point were used instead. Exenatide was measured under the same conditions.

### 1.13 - *Chronic exenatide long-term treatment study in obese/diabetic mice*

Eight-week-old male mice were randomly divided into 7 groups groups (10 mice per group) and housed 5 per cage in a controlled environment (room temperature of $23°C \pm 2°C$, 12 h daylight cycle) with free access to sterile food (AIN93Mi; Research diet) and sterile water. After a two-week acclimation period, mice underwent 8 weeks of HFD (60% fat and 20% carbohydrates (kcal per 100 g), D12492i, Research Diets, USA) (exenatide-treated groups) or a normal chow diet (Control). After this period, within the following 5-week treatment period, mouse body weight was recorded daily, and the glycemia was monitored once per week. Mice were treated daily at 4 pm (i) orally with exenatide in solution or encapsulated within RM LNC (500 μg/kg dose) (EXE RM LNC) or the corresponding concentration of unloaded RM LNC or (ii) subcutaneously with exenatide in solution (10 μg/kg) or as Byetta® (10 μg/kg) (marketed exenatide subcutaneous injection). Control groups (healthy and HFD) were treated orally daily with an equivalent volume of sterile MilliQ water. Mice were fasted once per week for 6 h prior to the glucose test. At the end of the treatment period, animals were anesthetized with isoflurane (Forene, Abbott, England), and blood was sampled from both the portal and the cava veins. After exsanguination, mice were euthanized by cervical dislocation. Subcutaneous adipose tissue, liver and spleen were precisely dissected, weighed, and immediately immersed in liquid nitrogen followed by storage at -80°C for further analysis or preserved in 4% paraformaldehyde (PFA) for histological analysis (liver). Effects on body composition and adipose tissue were evaluated by the weights of subcutaneous (SAT), epididymal (EAT), visceral adipose tissues (VAT) and brown adipose tissues (BAT) (mg). Levels of glucose and gastrointestinal hormones involved in food intake and body weight, including total GLP-1 (ELISA kit, Meso Scale Delivery, Gaithersburg, USA) and insulin (Ultrasensitive insulin ELISA, Mercodia, Uppsala, Sweden), were measured in peripheral blood and portal vein blood. Liver steatosis was visualized by oil red O staining. Liver tissue was embedded in Tissue-Tek Optimal Cutting Temperature compound (Sakura Europe, Leiden, Netherlands) and flash-frozen in cold isopentane. Five-micrometer-thick tissue sections were stained with oil red O staining for lipid content analysis. Five high-magnification fields (20x) were analyzed per mouse. Quantification of the mean droplet area was performed using ImageJ software (Version 2.0.0-rc-69/1.52i, National Institutes of Health, Bethesda, Maryland, USA). The general morphology of the liver was assessed by hematoxylin and eosin (H&E)-stained sections. For the real-time quantitative PCR (qRT-PCR) analysis, the total RNA was isolated from tissues using TriPure reagent (Roche). Complementary DNA was prepared by reverse transcription of 1 $\mu$g total RNA using a Reverse Transcription System kit (Promega, Madison, Wisconsin, USA). Real-time PCR was performed with a CFX96 real-time PCR system and CFX Manager 3.1 software (Bio-Rad, Hercules, California, USA) using GoTaq® qPCR Master Mix (Promega, Madison, USA) for detection according to the manufacturer's instructions. Ribosomal protein L19 (Rpl19) was chosen as the housekeeping gene. All samples were run in duplicate in two 96-well reaction plates, and data were analyzed according the 2-$\Delta\Delta$CT method. The identity and purity of the amplified product were assessed by melting curve analysis at the end of the amplification. Primer sequences for the targeted mouse genes are presented in **Table 3.**

**Table 3:** Primer sequences for gene expression analyses by RT-qPCR

| SEQ ID NO: | | Sequence |
|---|---|---|
| 1 | Rpl19 forward | GAAGGTCAAAGGGAATGTGTTCA |
| 2 | Rpl19 reverse | CCTTGTCTGCCTTCAGCTTGT |
| 3 | F4/80 forward | TGACAACCAGACGGCTTGTG |
| 4 | F4/80 reverse | GCAGGCGAGGAAAAGATAGTGT |
| 5 | Cd11c forward | ACGTCAGTACAAGGAGATGTTGGA |
| 6 | Cd11c reverse | ATCCTATTGCAGAATGCTTCTTTACC |
| 7 | Mcp1 forward | GCAGTTAACGCCCCACTCA |
| 8 | Mcp1 reverse | TCCAGCCTACTCATTGGGATCA |
| 9 | Tnfa forward | TCGAGTGACAAGCCTGTAGCC |
| 10 | Tnfa reverse | TTGAGATCCATGCCGTTGG |
| 11 | Fasn forward | CAGGCCCCTCTGTTAATTGG |
| 12 | Fasn reverse | TCCAGGGATAACAGCACCTT |
| 13 | Pparg forward | CTGCTCAAGTATGGTGTCCATGA |
| 14 | Pparg reverse | TGAGATGAGGACTCCATCTTTATTCA |
| 15 | Cpt1a forward | AGACCGTGAGGAACTCAAACCTAT |
| 16 | Cpt1a reverse | TGAAGAGTCGCTCCCACT |

Total lipids were measured after extraction with chloroform-methanol according to a modified Folch method (Biol Chem. 1957; 226, 497-509), as previously described (Everard et al., Nat Commun. 2019; 10, 457). Triglyceride and cholesterol concentrations were measured using a kit coupling an enzymatic reaction and spectrophotometric detection of the final product (Diasys Diagnostic and System, Holzheim, Germany). All samples were run in duplicate.

_1.14 - Statistical analysis_

GraphPad Prism 7 program (CA, USA) was used to perform the statistical analyses. For all analyses and for each group, any exclusions were supported by the use of the Grubbs test for outlier detection. Values were normalized by log-transformation when variances were significantly different between groups before conducting the analysis. Two-way or one-way ANOVA followed by Tukey's post hoc test was applied for comparisons among multiple groups. If the variance differed significantly between groups even after normalization, a nonparametric test was performed. The results are expressed as the mean $\pm$ standard error of the mean (SEM). A difference of P<0.05 was considered statistically significant.

## 2. Results and discussion

### 2.1 - Exenatide is successfully encapsulated and preserved within lipid-based lipid nanocapsules

It was recently discovered that lipid-based lipid nanocapsules (LNC) triggered endogenous GLP-1 secretion *in vivo* in mice when presenting a size of approximately 200 nm (Xu et al., Mol Pharm. 2018; 15, 108-115). It was not known whether synergizing the biological effect of LNC together with the pharmacological effects of an encapsulated GLP-1 analog could be feasible. Hence, as a proof of concept, the GLP-1 analog exenatide (EXE) was selected as the incretin mimetic to be encapsulated within LNC, which is a hydrophilic molecule. To assess this possibility, it was important to encapsulate exenatide within the liquid lipid inner core of the lipid nanocapsules while preserving the physico-chemical characteristics of the nanocarrier, responsible for the *in vivo* biological effect (*e.g.*, triggering endogenous GLP-1 secretion) observed with the lipid nanocapsule per se (Xu et al., Mol Pharm. 2018; 15, 108-115). LNC were prepared following a phase inversion process (Heurtault et al., Pharm Res. 2002; 19, 875-880). Compared to the previously described procedure used to prepare LNC (Xu et al., Mol Pharm. 2018; 15, 108-115), the inner oily core was replaced with a mixture of caprylic/capric acid triglycerides (Labrafac® WL 1349) and oleic acid mono-, di- and triglycerides (Peceol®). This variation allowed reducing the temperature cycles of progressive heating/cooling from 60°C to 90°C, to 50°C to 67°C, which enabled the incorporation of EXE into LNC. Prior to its encapsulation into LNC, EXE was entrapped within reverse micelles (RM) using a surfactant-oil combination composed of sorbitan oleate (Span® 80) and a mixture of caprylic/capric acid triglycerides (Labrafac® WL 1349) (Anton et al., Int J Pharm. 2010; 398, 204-209). These EXE RM were then incorporated into the formulation during the last cycle of the LNC preparation process. The final composition of the formulation is described in **Table 1** above, and the physico-chemical characterization is detailed in **Table 4.**

**Table 4:** Physicochemical characterization of EXE-loaded or unloaded RM LNC (n=9)

| Formulation | Mean size (nm) | PDI | Zeta potential (mV) | EE (%) |
|---|---|---|---|---|
| EXE RM LNC | 224.4 $\pm$ 3.2 | 0.185 $\pm$ 0.013 | -3.060 $\pm$ 0.400 | 84.95 $\pm$ 3.76 |
| RM LNC | 215.7 $\pm$ 3.3 | 0.165 $\pm$ 0.012 | -2.993 $\pm$ 0.290 | - |

The mean particle size of both EXE RM LNC and lipid nanocapsules encapsulating RM without the peptide (RM LNC) was approximately 220 nm. The small PDI index (PDI<0.2) indicated the homogeneity of the resulting lipid nanocapsules in terms of size distribution. In addition to the size and PDI, there was also no significant influence on the surface charge of the lipid nanocapsules after exenatide encapsulation (P>0.05). Notably, EXE RM LNC exhibited an entrapment efficiency of approximately 85%.

Lipid nanocapsules remained stable and prevented exenatide degradation *in vitro* in biomimetic gastrointestinal fluids **(Fig. 1)**. The stability of the lipid nanocapsules was confirmed in five biomimetic media, including fasted- and fed-state simulated gastric or intestinal fluids (FaSSGF with or without pepsin, FaSSIF, FeSSIF and FeSSIF-v2, respectively) **(Fig. 1A-E)**. These results confirmed that the newly developed lipid nanocapsules retained the same gastro-resistant properties as previously described conventional lipid nanocapsules (Xu et al., Mol Pharm. 2018; 15, 108-115; Roger et al., Int J Pharm. 2009; 379, 260-265). The *in vitro* exenatide release profile was further evaluated in gastric medium (FaSSGF without pepsin, pH 1.6) and intestinal medium (FaSSIF, pH 6.5) **(Fig. 1A-F)**. Exenatide was progressively released from the RM LNC over a 6 h period, reaching a cumulative exenatide release of 60% after this time in FaSSIF, which was undetectable in FaSSGF **(Fig. 1F)**.

### 2.2 - RM LNC induce GLP-1 secretion both in vitro and in vivo and increase EXE blood levels in normoglycemic mice

First, the ability of RM LNC on their ability to trigger GLP-1 secretion was investigated *in vitro* in both murine L cells (GLUTag cells) and in human L cells (NCI-H716) **(Fig. 2A)**. RM LNC of particle size approximately 220 nm were able to trigger endogenous GLP-1 secretion in both *in vitro* models **(Fig. 2A)**. Importantly, although an additional liquid lipid was incorporated in the inner core of the RM LNC, the stimulation of GLP-1 secretion was comparable to those previously described for lipid nanocapsules containing a caprylic/capric acid triglycerides core (Xu et al., Mol Pharm. 2018; 15, 108-115). Therefore, these data confirmed that by preserving the nanosystem external physico-chemical properties (*e.g.*, surface charge), it was possible to maintain the ability of the nanocarrier to induce GLP-1 secretion.

Whether RM LNC could trigger GLP-1 secretion *in vivo* in normoglycemic mice was further investigated **(Fig. 2B).** Notably, the pharmacological effect was preserved *in vivo* because following the oral administration of RM LNC, it was observed that the GLP-1 levels were increased up to approximately 3-fold **(Fig. 2B).** Therefore, the pharmacological effect of the nanocarriers was reproduced *in vitro* in both cell lines irrespective of the nature of the cells (murine or human) and *in vivo* in normoglycemic mice.

To validate the hypothesis that RM LNC not only trigger the endogenous secretion of GLP-1 but also act as a dual-action as nanocarrier for the oral delivery of incretin mimetic peptides, the ability of the nanocarriers to increase the absorption of these peptides was assessed. For this purpose, the exenatide plasma concentration was measured upon oral administration as a solution in water or encapsulated within RM LNC (500 $\mu$g/kg exenatide dose). Exenatide plasma levels were higher when encapsulated within RM LNC than when delivered as free exenatide in solution **(Fig. 3).** Altogether, these data confirmed the hypothesis regarding the efficacy of RM LNC at both enhancing endogenous GLP-1 levels and increasing EXE plasmatic levels, thus enabling the peptide's absorption.

In addition, no evidence of EXE RM LNC cytotoxicity could be observed in human intestinal epithelial Caco-2 cells after either increasing drug concentrations 0.5-10 mg/mL **(Fig. 4A) or** the nanoparticle concentration 2-18 mg/mL) **(Fig. 4B).** The cytotoxicity of RM LNC in GLUTag and NCI-H716 cells is described in **Fig. 4C** and **Fig. 4D,** respectively.

### *2.3 - The combination of endogenous GLP-1 release with increased exenatide plasma levels improves glycaemia in diabetic mice*

The therapeutic relevance regarding the combination of nanocarrier-mediated endogenous GLP-1 levels with increased EXE plasma concentration was evaluated in a diet-induced obese/diabetic mouse model. Administration of EXE RM LNC was performed in a murine high-fat diet (HFD)-induced T2DM model (C57BL/6J mice) following acute treatment (one single administration). First, it was confirmed that the HFD mice were markedly hyperglycemic and hyper-insulinemic in the fasted state and displayed a strong degree of insulin resistance (*e.g.*, index of insulin resistance).

A 500 $\mu$g/kg exenatide dose was administered once orally (free and encapsulated within RM LNC) and equivalent concentrations of RM LNC or water 60 min prior to an oral glucose administration (2 g/kg). Strikingly, we found that EXE RM LNC treatment completely normalized the glycemia, as glycemia in these mice followed the same profile throughout the overall oral glucose challenge as that observed in lean normoglycemic control mice **(Fig. 5).** Conversely, the glucose levels measured in the EXE-treated mice remained similar to those in the HFD-fed mice at 30 min and then remained higher than those in the EXE RM LNC-treated mice until 90 min **(Fig. 5).** EXE RM LNC were able to significantly decrease plasma glucose levels and glucose area under the curve (AUC) **(Fig. 6).** Importantly, it was observed that total GLP-1 levels were significantly increased in both RM LNC- and EXE RM LNC-treated groups compared with the control groups (Control and HFD), confirming the ability of the nanosystem per se to stimulate GLP-1 release under pathological conditions. However, only EXE RM LNC significantly reduced the insulin resistance index compared with the HFD group **(Fig. 7).**

Interestingly, RM LNC alone had an effect on lowering blood glucose levels compared with untreated HFD mice. However, this effect was not sufficient as per reducing the hyperglycemia. A pharmacokinetic study measuring exenatide levels in HFD mice confirmed that exenatide blood levels were significantly increased when orally administered within RM LNC (EXE RM LNC) compared with exenatide in solution (relative bioavailability 4.32% with LNC, P<0.001). This relative bioavailability can be considered a high value and a great improvement, considering known peptides having transitioned to the late phase of development with an estimated 0.5-1% bioavailability. The calculated pharmacokinetic parameters are summarized in **Table 5.**

**Table 5:** Pharmacokinetic parameters of the in vivo study in obese/diabetic mice for s.c. exenatide (50 $\mu$g/kg), oral exenatide (500 $\mu$g/kg) and oral EXE RM LNC (500 $\mu$g/kg). P values were significantly different ([a]P<0.01 and [b]P<0.001) according to a one-way ANOVA followed by Tukey's post hoc test. Significance is expressed compared to the exenatide oral group

| Formulation | $C_{max}$ (ng/mL) | $T_{max}$ (h) | $AUC_{0-8}$ (ng · h/mL) | $t_{1/2}$ | FR (%) |
|---|---|---|---|---|---|
| Exenatide s.c. | 10.27 ± 6.82[a] | 1.5 | 27.30 ± 12.46[b] | 1.38 ± 0.36 | 100[b] |
| EXE LNC oral | 8.73 ± 4.53[a] | 0.5 | 11.79 ± 2.73[b] | 1.68 ± 0.35 | 4.32 ± 0.95[b] |
| Exenatide oral | 0.86 ± 0.93 | 0.5 | 3.71 ± 0.93 | - | 1.36 ± 0.34 |

We observed a different exenatide pharmacokinetic profile (*e.g.*, different $C_{max}$, AUC, $T_{max}$) in normoglycemic **(Fig. 3)** versus HFD mice **(Fig. 8).** Taking together the increased exenatide blood levels and the increased endogenous GLP-1 levels, these data serve as a strong proof of concept regarding the effectiveness of the developed nanosystem in ameliorating T2DM symptoms.

The impact of EXE versus EXE RM LNC was further investigated in mice treated for 8 weeks and 10 weeks with HFD, since this model is a stronger model of diet-induced diabetes and metabolic disorders in mice. Equivalent results regarding the efficacy of EXE RM LNC on the reduction of blood glucose levels during OGTT were obtained regardless of the chronicity of the disease **(Fig. 9A-B).**

*2.4 - Chronic EXE RM LNC long-term treatment improves glucose metabolism in obese and diabetic mice*

To evaluate the impact of chronic and long-term treatment with EXE RM LNC on glucose metabolism, obese/diabetic-prone mice were subjected to 8 weeks of HFD followed by 5 weeks of HFD with daily administration of 500 μg/kg exenatide (oral) (EXE RM LNC) or equivalent amounts of unloaded lipid nanocapsules (RM LNC) or free exenatide (EXE) in solution or water. To compare the impact of this experimental treatment with existing treatment strategies, a group treated with a marketed subcutaneous form of exenatide (Byetta®) was also included. The oral administration of exenatide was compared to the subcutaneous injection (s.c.) of 10 μg/kg exenatide in solution or as Byetta®.

After 5 weeks of daily treatment, mice were sacrificed, and blood was drawn from both the portal and the cava vein. Interestingly, after 5 weeks of treatment, only the EXE RM LNC were able to decrease plasma glucose levels to reach levels comparable to that of the control group **(Fig. 10A-B).** It is noteworthy to mention that EXE RM LNC-treated mice had significantly lower plasma glucose levels than the RM LNC-treated mice. Plasma glucose levels were also significantly lower in the RM LNC group than in the EXE-treated. Therefore, these data demonstrate the synergistic effect provided by EXE RM LNC on glucose homeostasis. This effect is likely due to the combination of the endogenous GLP-1 secretion triggered by RM LNC and elevated exenatide plasma levels. Additionally, EXE RM LNC-treated and subcutaneously treated mice exhibited comparable insulin levels as the control group **(Fig. 11).** Although this reduction compared with the HFD group did not reach statistical significance when the data were analyzed by the Kruskal-Wallis followed by Dunn's post hoc test, a significant difference was found when analyzed by the Mann-Whitney test (P=0.04 for EXE RM LNC versus HFD).

*2.5 - Chronic treatment with EXE RM LNC decreases diet-induced steatosis in obese/diabetic mice*

We found that the liver weight was only significantly lower in the EXE RM LNC-treated group after 5 weeks of treatment (13 weeks of HFD feeding) compared with the HFD-fed group **(Fig. 12).** Histological analysis after oil red O staining revealed a marked decrease in hepatic steatosis, as evidenced by lower hepatic lipid accumulation and by fewer and smaller lipid droplets in EXE RM LNC-treated mice than in HFD mice (data not shown).

Despite the minor impact on triglyceride levels, hepatic total lipid content and cholesterol levels were comparable between EXE RM LNC-treated and subcutaneously treated mice and were significantly lower than those in the HFD group **(Fig. 13A-C).**

It is of the utmost importance to note that the instant approach using EXE RM LNC was more efficient in decreasing liver weight than all the other treatments and was efficient as the marketed drug by the typical administration route (s.c.), thereby clearly showing the better effects on glucose parameters and a noninferiority on liver markers compared with the current subcutaneous approach.

In addition to the biochemical and histological analysis, key markers associated with infiltration/recruitment of immune cell populations (F4/80, Cd11c, Mcp1), inflammation (Tnfa) and lipid metabolism (Fasn, Pparg, Cpt1a) were analyzed by quantitative PCR both in the liver and in visceral adipose tissue (VAT). In the liver, Cd11c and Mcp1 mRNA expression in EXE RM LNC-treated mice was significantly lower than that in the HFD group (P=0.05 and P=0.0355, respectively), and the same effect was observed in the Byetta®-treated group (P=0.0015 and P=0.0007, respectively).

Visceral fat mass is considered a risk factor for developing liver diseases and insulin resistance (Perseghin, Diabetes Care. 2011; 34 Suppl 2, S367-370; Lebovitz & Banerji, Diabetes Care. 2005; 28, 2322-2325). It was found that both the EXE RM LNC- and Byetta®-treated groups exhibited less fat than in HFD-fed mice, and there was no significant difference in the amount of fat in these groups from that in the untreated control group **(Fig. 14A-E).** Their weights were highly significantly different when analyzed by the Mann-Whitney test (P=0.0057 for EXE RM LNC versus HFD and P=0.0004 for Byetta® versus HFD). Interestingly, higher expression of F4/80 observed in the HFD group was significantly downregulated only in EXE RM LNC-treated mice following ANOVA. No significant effects were observed for the other markers (Cd11c, Mcp1, Fasn, Pparg, Tnfa), when compared with the HFD group.

*2.6 - Discussion*

Despite numerous ongoing efforts, the transformation of injectable therapies for T2DM into oral drug delivery strategies remains a challenge. As a result, current treatments with GLP-1 analogs on the market are still administered exclusively subcutaneously. Studies aimed at providing with alternative drug delivery systems for peptides are of the utmost importance to fully take advantage of the potential of the oral route of administration. However, current state-of-the-art strategies for oral peptide delivery use the delivery system merely as a vehicle, and none of them have explored the possibility that the carrier could have additional therapeutic effects on the final formulation. In the context of incretin-based diabetes disease therapy, the enhancement of endogenous GLP-1 secretion represents a novel alternative treatment that more closely resembles the physiology of the peptide. Here is provided a lipid-based drug delivery system, namely lipid nanocapsules, having dual therapeutic effects on (i) the delivery of encapsulated synthetic GLP-1 analogs and (ii) the stimulation of the endogenous GLP-1 secretion.

One major challenge in the development of this delivery system was to encapsulate a hydrophilic peptide within a lipid core while preserving the physico-chemical properties of the lipid nanocapsule. For this purpose, exenatide was first entrapped within reverse micelles, which in turn were themselves included into the inner lipid core of lipid nanocapsules. This strategy enabled to encapsulate a GLP-1 mimetic within lipid-based nanocarriers without altering their structure and retaining their efficacy for L cell activation, thus triggering endogenous GLP-1 secretion.

The ability of exenatide-loaded lipid nanocapsules to induce GLP-1 secretion was confirmed *in vitro,* both in murine and human L cells, and *in vivo* in normoglycemic mice, **(Fig. 2A-B).** Additionally, we conducted a pharmacokinetic study confirming the absorption of exenatide into systemic circulation **(Fig. 3).** Evidences of the stability of the formulation, the ability of the nanosystem to preserve peptide integrity, its ability to increase GLP-1 levels *in vivo* while enabling the absorption of the peptide into systemic circulation, and finally the efficacy of the formulation in the pathological context in a HFD-induced obese/diabetic mouse model are provided herein.

The utility of our dual-action nanosystem for improving glycemia *in vivo* in obese/diabetic mice following an acute treatment was first evaluated. After a single acute administration followed by an OGTT test, EXE RM LNC-treated mice normalized blood glucose levels, which were comparable to those of the control group. Although both empty and EXE-loaded lipid nanocapsules presented increased GLP-1 levels compared with untreated groups or the groups treated with EXE in solution, it was observed that only EXE RM LNC-treated mice exhibited a significant decrease in the insulin resistance index. A pharmacokinetic analysis in obese/diabetic mice confirmed that EXE RM LNC increased exenatide bioavailability to more than 4%. These data provide evidences of the effectiveness of a dual-action drug delivery nanosystem in ameliorating glycemia by combining both increased endogenous GLP-1 levels and increased peptide bioavailability.

To demonstrate that this nanosystem represents an alternative to current subcutaneous strategies for the delivery of incretin peptides in the treatment of T2DM, chronic/long-term treatment consisting of a five-week daily administration protocol was performed. The effect of the nanosystem on glucose homeostasis and lipid metabolism was evaluated. After 5 weeks of treatment, EXE RM LNC-treated mice exhibited normalized plasma glucose levels comparable to those of untreated control mice, along with decreased insulin levels. Therefore, the beneficial effects observed are not limited to the acute effect of the nanocarriers but are also translated to a therapeutic effect on oral glucose tolerance.

Altogether, the exenatide-loaded lipid nanocapsules were effective at reducing both hyperglycemia and hyperinsulinemia.

It is worth noting that this approach led to comparable results regarding glucose homeostasis to those observed for the current marketed drug that is administered subcutaneously. Therefore, these results demonstrate the noninferiority of this approach together with the benefit of administration by the oral route for chronic treatments.

These data also point to a strong trend towards lower key markers associated with infiltration-recruitment of immune cell populations (macrophages, dendritic cells) and inflammatory markers in both the liver and visceral adipose tissue, again highlighting the beneficial effects of this approach on such markers. F4/80 is a marker of inflammatory cell infiltration (mature macrophages), whereas Cd11c, Mcp1 and Tnfa are known to reflect the M1 macrophage phenotype during obesity- associated inflammation. It is important to note that EXE RM LNC were the only treatment that significantly reduced the gene expression of F4/80 in the visceral adipose tissue. In obesity, macrophage infiltration in adipose tissue leads to chronic inflammation, which is considered a triggering factor for insulin resistance and diabetes (Weisberg et al., Clin Invest. 2003; 112, 1796-1808). However, the ablation of macrophages in mice was associated with a normalization of glucose homeostasis (Hernandez et al., Cell Metab. 2014; 20, 499-511). From a mechanistic point of view, we previously showed that RM LNC increase the secretion of GLP-1 and, likely, the co-peptide GLP-2, which has been shown to reinforce the gut barrier function, thereby reducing bacterial compound translocation, inflammation and steatosis in obese rodents (Cani et al., Gut. 2009; 58, 1091-1103).

Without wishing to be bound by a theory, the daily oral gavage may stimulate the production of the gut peptides throughout the day and, therefore, may contribute to maintaining a better metabolic profile in the HFD-fed mice, with either EXE RM LNC or the RM LNC. In any case, a daily chronic administration is sufficient to improve metabolism and even normalize other markers. Finally, lipid nanocapsules may modulate the activity of DPP-IV, thus preserving increased circulating total GLP-1 levels in the body.

**Example 2: Working example 2**

## 1. Materials and methods

### 1.1 - Materials

Exenatide (exenatide acetate) (EXE) was purchased from Bachem® (Bubendorf, Switzerland). Labrafac® WL 1349 (caprylic/capric acid triglycerides) and Peceol® (oleic acid mono-, di- and triglycerides) were kindly provided by Gattefossé (Saint-Priest, France). Lipoid® S 100 (soybean lecithin at 94% of phosphatidylcholines) was a gift from Lipoid GmbH (Ludwigshafen, Germany). Solutol® HS15 (mixture of free PEG 660 and PEG 660 12-hydroxystearate, Mw 870 Da) and Span® 80 (sorbitan oleate) were purchased from Sigma-Aldrich (St. Louis, USA). The 1,2-distearoyl-sn-glycero-3-phosphoethanolamine-methoxylpoly(ethylene glycol)2000 (DSPE-PEG$_{2000}$-CH$_3$), 1,2-distearoyl-sn-glycero-3-phosphoethanolamine-N-[poly(ethylene glycol)-2000]-propionate (DSPE-PEG$_{2000}$-CH$_2$-CH$_2$-COOH) were obtained from Nanosoft Polymers (Winston-Salem, USA). Lecithin, sodium chloride (NaCl), saponin, pepsin, Triton-X 100, sodium taurocholate and dimethyl sulfoxide (DMSO), 3-(4,5- dimethyl-thiazol-2-yl)-2,5-diphenyl tetrazolium bromide (MTT) were purchased from Sigma-Aldrich (St. Louis, USA). Total GLP-1 (ver.2) and active GLP-1 assay kits were purchased from Meso Scale Discovery (Maryland, USA). The Exendin-4 enzyme immunoassay kit was purchased from Phoenix Europe GmbH (Karlsruhe, Germany). The Ultrasensitive Mouse Insulin ELISA Kit was purchased from Mercodia AB (Uppsala, Sweden). Matrigel™ was obtained from BD Bioscience (Belgium). Dipeptidyl peptidase IV (DPP-IV) inhibitor was purchased from Millipore (St. Charles, USA). Dulbecco's Modified Eagle's Medium (DMEM)-GlutaMAX (5.5 Mm glucose), fetal bovine serum (FBS), penicillin-streptomycin (P/S), trypsin (0.25%)-EDTA (0.02%) and phosphate-buffered saline (PBS) were also used and purchased from Thermo Fisher Scientific (Invitrogen, Belgium). DiD (DiIC18(5) solid (1,1'-ioctadecyl-3,3,3',3'-tetramethylindodicarbocyanine,4-chlorobenzenesulfonate salt)) was obtained from Thermo Fisher Scientific (Invitrogen, UK). All chemical reagents utilized in this study were of analytical grade.

### 1.2 - Preparation and characterization of non-targeted and targeted nanoparticles

#### 1.2.1 - Preparation of non-targeted and targeted lipid-based nanosystems

The non-targeted lipidic nanosystem (EXE RM LNC) containing exenatide-encapsulated reverse micelles (EXE RM) and modified lipid nanocapules (LNC) was prepared as described in example 1. First, exenatide-encapsulated reverse micelles (EXE RM) were prepared by stirring under high speed a mixture consisting of Span® 80 (surfactant) and Labrafac® WL 1349 (oil) with a ratio of 1:5 w/w. During stirring, 50 µL of drug solution (exenatide 30 mg/mL in MilliQ water) was dripped into the mixture. Second, LNC were produced by the phase inversion process, with slight modifications. The mixture of 769.5 mg Labrafac® WL 1349, 85.5 mg Peceol®, 13.4 mg Lipoid® S100, 120 mg Solutol® HS15, 50 mg sodium chloride (NaCl) and 1.025 mL of MilliQ water was stirred at 200 rpm for 5 min. Three progressive heating/cooling cycles (between 50°C and 67°C) were conducted. In the last cycle, 500 µL of pre-heated EXE RM was added to the mixture when the temperature was approximately at 3 °C above the phase inversion zone (PIZ; 59 to 61.5°C). Finally, 2.5 mL of cold water was added to reach the PIZ temperature under high-speed stirring for 2 min. The blank non-targeted nanosystems (RM LNC) were produced using the same protocol without adding the drug solution.

#### 1.2.2 - Preparation of DiD-labeled non-targeted and targeted lipid-based nanosystems

DiD-labeled RM LNC were prepared by adding 225 µg of DiD to the mixture. Namely, 45 µL of DiD (5 mg/mL in ethanol) was added to the vial and then heated in a water bath until the organic solvent was completely evaporated. Subsequently, Labrafac® WL 1349 was added to the same vial and mixed with DiD in the water bath until the DiD was completely dissolved in the oil phase. The mixture was then cooled down to room temperature (about 20°C), other components were added and the preparation process was continued as above described.

#### 1.2.3 - Preparation of PEGylated reverse micelle-loaded lipid nanocapsules with or without propionate

PEGylated reverse micelle-loaded lipid nanocapsules with or without propionate (RM LNC PEG or RM LNC PEG-PRO, respectively) were produced by incubating DSPE-PEG$_{2000}$-CH$_3$ or DSPE-PEG$_{2000}$-CH$_2$-CH$_2$-COOH, respectively, with RM LNC at a concentration of 5 mg/mL, under gentle stirring at 37°C for 4 h. During the incubation, the suspension was vortexed every 15 min and then quenched in an ice bath for 1 min.

### 1.3 - Quantification of exenatide

The exenatide encapsulated within PEGylated RM LNC was quantified by high-performance liquid chromatography (HPLC, Shimadzu, Japan) using a gradient method as described in example 1 (see 1.4).

*1.4 - Characterization of NPs*

The mean diameter, polydispersity index (PDI), zeta potential and drug encapsulation efficiency (EE, %) of PEGylated RM LNC NPs were determined as disclosed in example 1 (see 1.5).

*1.5 - Nanocapsule stability and drug release in stimulated gastrointestinal fluids*

1.5.1 - PEGylated nanocapsule stability in stimulated gastrointestinal fluids

The *in vitro* stability of PEGylated EXE RM LNC, with or without propionate as a ligand, was evaluated as disclosed in example 1 (see 1.6).

1.5.2 - *In vitro* drug release studies

Drug release from PEGylated EXE RM LNC was evaluated in FaSSGF in the absence of pepsin and in FaSSIF media for 2 h and 6 h, respectively as disclosed in example 1 (see 1.7).

*1.6 - In vitro cell studies*

1.6.1 - Cell cultures

The intestinal murine L cell line GLUTag was kindly provided by Dr. Daniel Drucker (University of Toronto, Canada). GLUTag cells were used from passages 17 to 25. Cells were grown in DMEM GlutaMAX (5.5 mM glucose) supplemented with 10% (v/v) inactivated FBS and 1% (v/v) P/S (complete DMEM medium) at 37 °C with 5% $CO_2$ supply. Cells were passaged every 4-5 days.

1.6.2 - Cytotoxicity studies

*In vitro* cytotoxicity studies of unloaded PEGylated RM LNC nanocapsules were performed in GLUTag cells using nanoparticle concentrations calculated previously using the 3-(4,5-dimethylthiazol-2-yl)-(2,5-diphenyltetrazolium bromide) (MTT) colorimetric assay (Xu et al. Mol Pharm. 2018; 15:108-115). GLUTag cells ($5 \times 10^4$ cells per well) were seeded on Matrigel™-coated (10 μL/mL of medium) 96-well plates.
After washing the plates with prewarmed PBS buffer (x3), 100 μL of the nanoparticle suspension at increasing nanocapsule concentrations (0.5-10 mg/mL) was dispersed in DMEM GlutaMAX medium (without FBS) and co-incubated with GLUTag cells at 37 °C for 2 h. After incubation, the supernatants were replaced with 100 μL of 0.5 mg/mL MTT for 3 h. The purple formazan crystals were dissolved in 200 μL of DMSO for absorbance determination at 560 nm using a MultiSksan EX plate reader (Thermo Fisher Scientific, USA). Cells with Triton-X 100 (100% dead) and cells with culture medium (100% alive) were considered positive and negative controls, respectively. Tests were performed in triplicate.

1.6.3 - *In vitro* GLP-1 secretion

GLUTag ($1.8 \times 10^5$ cells per well) were seeded onto Matrigel™-coated 24-well cell culture plates and allowed to adhere for 24 h. The next day, the plate was gently washed using prewarmed PBS. The GLUTag cells were then co-incubated with DMEM GlutaMAX without FBS or unloaded PEGylated NPs (RM LNC nanocapsules with or without ligand). The media contained DPP-IV inhibitor at a final concentration of 50 μM (Millipore®, St. Charles, MO, USA). To test the effect of different nanoparticle concentrations on GLP-1 secretion, we used 0.5-2 mg/mL nanocapsule and 0.1-3 mg/mL nanoparticle concentrations, respectively. The protocole is described in example 1 (see 1.9).

*1.7 - In vivo studies*

1.7.1 - Animals

All protocols involving animal studies were approved by the Université catholique de Louvain ethical committee on animal experiments (20 18/UCL/MD/45; laboratory agreement LA1230418) and conducted in accordance with the Belgian legislation regarding the protection of laboratory animals (Royal Decree of 29 May 2013).

1.7.2 - Total GLP-1 secretion of ligand-conjugated and non-conjugated PEGylated NPs in normoglycemic mice

To test PEGylated RM LNC (with or without propionate), C57BL/6J male mice (20-25 g, 10 weeks; Janvier Laboratories, France) were randomly divided into four groups (8 mice each), and treated with approximately 1.62 mg/g nanocapsule dose, as disclosed in example 1 (see 1.10).

1.7.3 - Nanocapsule distribution in the obese/diabetic mouse small intestine and colon

Male C57BL/6J mice (8 weeks old) were randomly divided into 4 groups (n=4 per group). After acclimation (2 weeks), the mice underwent 10 weeks of HFD feeding (rodent diet with 60% fat and 20% carbohydrates (in kcal %)) (D12492i, Research Diets, USA). Before the experiments, the mice were fasted overnight with free access to water. For evaluation of the nanocapsule distribution in the small intestine and colon, DiD-labeled nanocapsules, including RM LNC, PEGylated RM LNC and PEGylated RM LNC-PRO (=1.62 mg/g nanoparticle dose), were administered via oral gavage. Control mice were orally administered an equivalent volume of MilliQ water. Sections of duodenum, jejunum, ileum and colon were excised 1 h post-administration and subsequently flash frozen in Optimal Cutting Temperature compound (OCT). For all tissues, 6-$\mu$m-thick sections were cut using a Leica CM-3050-S cryostat. The tissue sections were briefly fixed in 4% paraformaldehyde for 5 min. After washing the PBS containing 0.02% polysorbate 20, the slides were mounted with VECTASHIELD Hard Set Mounting Medium containing DAPI. The samples were examined by CLSM using a Zeiss confocal microscope (LSM 150) to capture serial images. The data were analyzed using Axio Vision software (version 4.8).

1.7.4 - Mucus extraction from obese/diabetic mouse intestine

Untreated 10 week-HFD-induced diabetic mice were fasted overnight with free access to water. Prior to mucus extraction, the mice were sacrificed by cervical dislocation. The protocol below was adapted from Wang et al. (Bio-protocol. 2017; 7:e2394). Briefly, the duodenum and apical jejunum were dissected and placed in a Petri dish, following a very gentle flush with PBS to remove decal matter in the intestine. The syringe with a 19.5-gauge needle was used to deliver PBS gently though one open end of the intestinal segments. The intestinal segments were cut longitudinally using surgical scissors. Then, the mucus was scraped off the intestinal segments using a cell scraper (1.8 cm blade, Falcon®) and transferred to a clean tube. The isolated mucus was stored at -20°C for further mucus diffusion and mucus stability studies.

1.7.5 - Single particle tracking and confocal microcopy

DID RM LNC, DID RM LNC PEG and DID RM LNC PEG-PRO were diluted in water or small intestine mucus of mice and mixed by gentle stirring. A volume of 5 $\mu$L was placed on a microscopy glass slide sealed with an adhesive spacer (S24737, Secure-Seal™ Spacer, Thermo Fisher) and a cover glass (#1.5).

For single particle tracking, fluorescent signals were recorded using a spinning disk confocal microscope (Nikon Eclipse Ti, Tokyo, Japan) equipped with an MLC 400 B laser box (Agilent Technologies, Santa Clara, CA, USA), a Yokogawa CSU-X1 confocal spinning disk device (Andor, Belfast, UK), an iXon ultra EMCCD camera (Andor Technology®, Belfast, UK) and NIS Elements software (Nikon, Japan). A 100x oil immersion objective lens (Plan Apo VC 100x oil, 1.4 NA, Nikon, Japan) was used. A stage-top incubator (37°C, Tokai Hit) in combination with an objective lens heater (Biotechs) was used during imaging. Movies of 100 frames with a temporal resolution of 43.9 ms were recorded 5-10 $\mu$m above the coverslip. No further analysis could be performed due to either nanocapsules accumulation in distinct parts of the mucus or leakage of the fluorescent label so that individual nanocapsules could no longer be seen or analyzed.

The samples prepared for single particle tracking were also studied by laser scanning confocal microscopy. Images were recorded on a Nikon A1R HD Confocal laser scanning microscope with a 60x/1.27 Plan Apo IR Water immersion objective (SR Plan Apo IR AC, Nikon) using a galvano scanner. A wavelength of 637 nm (LU-N4 Laser Unit) was used for excitation of DiD. Fluorescence signals were detected with a Multi-Alkali PMT (A1-DUG-2 GaAsP Multi Detector Unit). A stack of 19.5 $\mu$m above the glass surface with a step size of 0.5 $\mu$m at Nyquist resolution was recorded and a maximum intensity projection was created for visualization. All imaging was performed at RT.

1.7.6 - Pharmacological studies in HFD-induced obese/diabetic mice

Male C57BL/6J mice (8 weeks old) were randomly housed (five mice per cage). After an acclimation period (2 weeks) with a normal chow diet (AIN93Mi, Research Diets®, USA), the mice were fed a HFD for 10 weeks. The cages were randomly divided into 5 groups (10 mice per group) before the experiments. The mice underwent an overnight fast prior to oral gavage with free drug solution (EXE) (dose: 500 $\mu$g/kg), non-PEGylated or PEGylated drug-loaded nanosystems (EXE RM LNC or EXE RM LNC PEG) (dose: 500 $\mu$g/kg), or the empty PEGylated nanosystem (RM LNC PEG) (dose:

equivalent nanocapsule concentrations to the other groups). The control HFD group was orally administered an equivalent volume of sterile water. An oral glucose tolerance test (OGTT) was performed 1 h after oral administration of the abovementioned formulations as in example 1. Briefly, glucose was orally administered (2 g/kg), and then blood glucose determined with a glucose meter (Accu Check, Roche, Switzerland), measuring the blood from the tip of the tail vein 30 min prior to glucose load (-30 min) and 0, 15, 30, 90 and 120 min after glucose administration. The plasma insulin and total GLP-1 levels were also tested in plasma from blood samples collected from the tail vein at -30 min and 15 min using ELISA kits (Mercodia, Uppsala, Sweden and Meso Scale Delivery, USA, respectively). The insulin resistance index was calculated by multiplying the AUC of both blood glucose and insulin during OGTT. At the end of the OGTT test, the mice were anesthetized with isoflurane (Forene, Abbott, England), and blood samples were collected from the portal vein. Active GLP-1 levels were tested by ELISA (Meso Scale Delivery, Gaithersburg, USA).

### 1.7.7 - Pharmacokinetics study in obese/diabetic mice

Male C57BL/6J mice (8 weeks old) were housed for 2 weeks of acclimation and randomly divided into two groups (10 mice per time point). Next, the mice underwent a HFD feeding for 10 weeks, as described in the previous section. All mice were fasted overnight with free access to sterile MilliQ water before oral gavage. Exenatide in solution and EXE RM LNC PEG were orally administered at a dose of 500 $\mu$g/kg. At predetermined time points (0, 0.5, 1, 1.5, 2, 4, 6 and 8 h), blood samples were collected from the tip of the tail vein, centrifuged (1,500 g, 10 min at 4°C) and subjected to plasma extraction followed by storage at -80°C until further analysis. Exenatide plasma concentrations were determined using ELISA kits (EK-070-94, Phoenix Europe GmbH, Karlsruhe, Germany).

### 1.7.8 - Long-term treatment in obese/diabetic mice with different oral administration frequencies

Male C57BL/6J mice (8 weeks old) were randomly divided into seven groups (10 mice per group) and housed at five per cage with free access to sterile food (AIN93Mi; Research Diet) (control diet) and sterile water. After 2 weeks of acclimation, the mice underwent 10 weeks of HFD or a normal control diet. After this period, the mice were treated with our formulations for an additional month, continuing with the HFD feeding (14 weeks of HFD in total). During this month, the mice were orally administered daily (D) or once every two days (T) at 4 pm (i) empty or drug-loaded PEGylated formulations (500 $\mu$g/kg dose) with daily treatment at the equivalent nanocapsule concentrations (RM LNC PEG-D or EXE RM LNC PEG-D), and (ii) non-PEGylated or PEGylated drug-loaded formulations (500 $\mu$g/kg dose) with treatment every other day (EXE RM LNC-T or EXE RM LNC PEG-T). The control groups (healthy and HFD) were orally administered an equivalent volume of sterile Milli-Q water daily. During this 4-week treatment period, the body weight of the mice was recorded daily, and glycemia was monitored once per week. Mice were fasted once per week for 6 h prior to the glucose test.

### 1.8 - Statistical analysis

The GraphPad Prism 8 program (CA, USA) was used for the data analyses. The Grubbs test for outlier detection in each group was performed prior to all analyses. Before preforming the analysis, when there was a significant difference in the variance among groups, log-transformation was used to normalize the values. Statistical analyses were conducted using a two-way or one-way ANOVA followed by Tukey's post hoc test for studies containing more than two groups, and the t-test or Mann-Whitney test for two groups. The nonparametric test was conducted when there was a significant difference in variance between groups even after normalization. Statistical significance was considered at $p < 0.05$. All data are expressed as the mean $\pm$ standard error of mean (SEM).

## 2. Results and discussion

### 2.1 - Preparation and characterization of PEGylated nanoparticles with propionate as a targeting moiety

To develop L cell-targeted lipid-based nanocapsules, DSPE-PEG$_{2000}$ was selected as a PEGylation linker to provide the nanocapsules with an enhanced capacity for mucus diffusion. The post-insertion method was selected to ensure the PEG chains were localized only on the surface rather than retained within the particle core. Both DSPE-PEG$_{2000}$-OCH$_3$ and DSPE-PEG$_{2000}$-CH$_2$-CH$_2$-COOH were used to provide specific targeting to L cell GPCRs present on their surface. The physicochemical properties of empty lipid nanocapsules were assessed before (RM LNC) and after the post-insertion of both DSPE-PEG$_{2000}$-OCH$_3$ and DSPE-PEG$_{2000}$-CH$_2$-CH$_2$-COOH (RM LNC PEG and RM LNC PEG-PRO, respectively) (**Table 6**).

Table 6: Physicochemical characterization of EXE-loaded or unloaded or DiD-labeled RM LNC with different modifications

| Formulation | Mean size (nm) | PDI | Zeta potential (mV) | EE (%) |
|---|---|---|---|---|
| RM LNC | 204.8 ± 6.1 | 0.176 ± 0.007 | -0.94 ± 0.12 | - |
| RM LNC PEG | 222.7 ± 11.1 | 0.159 ± 0.009 | -9.93 ± 0.55 | - |
| RM LNC PEG-PRO | 231.6 ± 10.0 | 0.131 ± 0.033 | -18.20 ± 1.40 | - |
| DiD RM LNC | 206.5 ± 4.6 | 0.208 ± 0.506 | -1.31 ± 0.12 | - |
| DiD RM LNC PEG | 221.8 ± 9.9 | 0.156 ± 0.044 | -9.39 ± 1.07 | - |
| DiD RM LNC PEG-PRO | 225.9 ± 2.43 | 0.123 ± 0.010 | -16.20 ± 0.36 | - |
| EXE RM LNC | 208.4 ± 6.04 | 0.155 ± 0.021 | -2.70 ± 0.23 | 84.46 ± 3.88 |
| EXE RM LNC PEG | 213.3 ± 6.68 | 0.176 ± 0.022 | -9.23 ± 0.31 | ≈100 |

Blank LNC presented an approximately 200-nm particle size and a narrow size distribution (PDI=0.18). After incubation of LNC with biofunctional polymers, it was observed an increase in size of ~ 18 nm and ~ 27 nm for RM LNC PEG and RM LNC PEG-PRO, respectively. The zeta potential values decreased significantly from -0.94 mV (RM LNC) to -9.93 mV (RM LNC PEG). The surface charge of RM LNC PEG-PRO decreased even more, reaching -18.20 mV in the presence of the fatty acid. A similar particle size and zeta potential were obtained after labeling the nanocarriers with DiD when compared to the respective empty nanocapsules (**Table 6**). Additionally, there was also no significant influence on the size and surface charge of the nanocapsules when encapsulating exenatide within PEGylated nanocapsules (EXE RM LNC PEG) (**Table 6**).

*2.2 - PEGylated NPs induce GLP-1 secretion both in vitro and in vivo*

The ability of unloaded, PEGylated and propionate-grafted RM LNC to induce GLP-1 stimulation *in vitro* were first investigated in GLUTag cells (murine L cells). The nanoparticle concentration ranged from 0.5 mg/mL to 2 mg/mL, after observing no evidence of cytotoxicity at concentrations below 4 mg/mL. As shown in **Fig. 15A-B,** all lipid-based formulations were able to significantly increase endogenous GLP-1 secretion *in vitro* in L cells regardless of the nanoparticle concentration (\*\*p<0.05).

It was further tested whether these nanocapsules could further strengthen and/or induce GLP-1 stimulation *in vivo* in normoglycemic mice. When orally administered to normoglycemic mice, both PEGylated lipid nanocapsules (RM LNC PEG and RM LNC PEG-PRO, respectively) and non-PEGylated lipid nanocapsules (RM LNC) significantly increased GLP-1 levels 60 min post-administration (\*p<0.05) (**Fig. 16**). Notably, RM LNC PEG increased GLP-1 levels up to ~ 8-fold at 60 min, whereas RM LNC PEG-PRO had the same effect as the original unmodified RM LNC, increasing GLP-1 secretion up to ~ 4-fold compared with the untreated control group. Furthermore, only RM LNC PEG prolonged this effect at 180 min (\*p<0.05) when compared to the control group. Although PEGylated NPs did not exert a significant effect *in vitro* when compared to non-PEGylated NPs (**Fig. 15A-B**), PEGylation could significantly improve and prolong the ability of the nanocarriers to induce GLP-1 secretion *in vivo.*

*2.3 - Nanoparticle distribution in the small intestine and colon of obese/diabetic mice*

The distribution of the nanocapsules was tracked in different segments of the gut after oral gavage *in vivo* in 10-week HFD-fed mice. The accumulation of DiD-labeled nanocapsules within the small intestine and the colon upon oral administration to HFD mice was followed by the means of fluorescent nanoparticles. All the fluorescent particles (DiD RM LNC, DiD RM LNC PEG and DiD RM LNC PEG-PRO) could be found in the duodenum, in which the DiD RM LNC PEG group showed the strongest red fluorescence (DiD) compared with the other groups.

To investigate a potential difference in mucus diffusion, single particle tracking was performed. For this purpose, particles were mixed with small intestinal mucus, placed in a custom-made glass chamber and visualized on a spinning disk microscope. However, it was not possible to determine the particle diffusion *in vitro* in small intestinal mucus of mice due to a strongly reduced amount of observed particles compared with the particles in water. With confocal microscopy, large structures with increased fluorescence intensity compared with mucus alone were observed for all formulations, indicating aggregation and/or binding to mucus constituents. Since the mobility of individual nanocapsules could not be investigated, it was not possible to conclude if there were any differences in net mobility between the different formulations.

DiD-labeled RM LNC were observed mainly in the duodenum and hardly in the jejunum 1 h post-administration. Interestingly, one hour after the treatment, only RM LNC PEG were able to pass through the intestinal epithelia in the jejunum, with a high level of red fluorescence observed in the epithelial basal layer compared with the other groups. Additionally, only RM LNC PEG were able to pass through the whole small intestine (duodenum, jejunum and ileum) and reach the colon.

## 2.4 - Pharmacological and pharmacokinetics studies in obese/diabetic mice

Prior to the evaluation of the efficacy of the formulation *in vivo,* the stability of the formulation was demonstrated *in vitro* in simulated gastric and intestinal fluids, and the release profile of exenatide was evaluated.

To determine the therapeutic efficacy of exenatide-loaded RM LNC PEG (EXE RM LNC PEG) on controlling the post-prandial hyperglycemia in type 2 diabetic mice, the pharmacodynamics profile was performed in 10 weeks HFD-fed mice. A single dose of the nanocapsules was administered prior to an oral glucose tolerance test (OGTT). The exenatide dose of 500 $\mu$g/kg for the free drug solution and drug-loaded formulations (including EXE in solution, EXE RM LNC and EXE RM LNC PEG, respectively), empty RM LNC PEG (equal volume as per drug-loaded nanocapsules), or an equivalent water volume was orally administered 60 min before an oral glucose challenge (glucose concentration: 2 g/kg). The time point of glucose administration corresponded to 0 h (**Fig. 17A**). The plasma glucose profile of drug solution-treated mice showed a similar trend as the HFD-fed mice that received water. Conversely, all the nanocapsule-treated groups, including EXE RM LNC, EXE RM LNC PEG and empty RM LNC PEG, could markedly lower blood glucose levels and the glucose area under the curve (AUC) (**Fig. 17A**). It is remarkable that empty RM LNC PEG had a similar efficacy for reducing plasma glucose levels and a similar AUC compared with exenatide-loaded RM LNC, indicating that the hypoglycemic effect achieved by the nanocarrier alone after PEGylation was comparable to non-PEGylated nanocarriers encapsulating the drug. Hence, the GLP-1 levels secreted by PEGylated nanocapsules (**Fig. 16**) were found to be therapeutically relevant regarding a glucose lowering effect. It is worth noting that, among all the tested formulations, only EXE RM LNC PEG orally treated mice showed significantly decreased plasma glucose levels throughout the overall OGTT test compared with untreated HFD mice.

Additionally, the total GLP-1 levels (**Fig. 17B**) were also significantly improved in all nanocapsule-treated groups compared with the control groups (*p<0.05). Although the increase in total GLP-1 levels in the EXE RM LNC group did not reach significant differences compared with the HFD group analyzed by the Kruskal-Wallis and Dunn's post hoc tests, significant differences were observed when the Mann-Whitney test was applied (p=0.008) (**Fig. 17B**). The active GLP-1 concentration measured in the portal vein (3 h post-administration of the formulation) compared with the untreated HFD mice was also significantly increased in EXE RM LNC PEG-treated mice after the OGTT test (*p<0.05) (**Fig. 17C**). Although there were no significant differences between the RM LNC PEG and HFD when analyzing the data by one-way ANOVA, significant differences between these groups were encountered by the Student's t-test (p=0.028). These data confirm that PEGylation was able to improve the ability of the nanosystems to stimulate GLP-1 release and prolong this effect under pathological conditions. No differences were observed among groups regarding insulin levels (**Fig. 17D**). However, EXE RM LNC PEG could dramatically reduce the insulin resistance index compared with the untreated diabetic mice (**Fig. 17E**). Significant differences between these groups were encountered by the Student's t-test (p=0.035 and p=0.023, respectively), despite no significant differences between EXE RM LNC and RM LNC PEG by one-way ANOVA. Strikingly, it was obtained a comparable result with unloaded PEGylated RM LNC when compared to EXE-loaded nanocapsules, further confirming the therapeutic relevance of the increased GLP-1 levels obtained through PEGylation.

To investigate the oral delivery of exenatide via PEGylated RM LNC, a pharmacokinetic study was performed to evaluate the oral absorption of exenatide in chronic diabetic mice (10 weeks of HFD; n=10 per point) after oral administration of a single dose of 500 $\mu$g/kg exenatide via drug solution or within RM LNC PEG (EXE RM LNC PEG) (**Fig. 17F**). A different pattern of exenatide absorption was observed in obese/diabetic mice after oral gavage of drug solution alone or EXE RM LNC PEG. When orally administered as a solution, the plasma concentration of exenatide remained unchanged throughout the testing period. In comparison, EXE RM LNC PEG elicited greater systemic absorption of the peptide after oral administration for 8 h, with a Tmax and Cmax of 1 h and 27.91 $\pm$ 1.22 ng/mL, respectively. One should note that a different exenatide pharmacokinetic profile (e.g., different Cmax, AUC, Tmax) was observed after oral administration of EXE RM LNC PEG versus EXE RM LNC, indicating that PEGylation extended the circulation time of the formulation (t1/2 of the drug-loaded formulation prolonged from 1.68 $\pm$ 0.35 to 5.69 $\pm$ 1.02 h) and then increased the systemic absorption of the encapsulated peptide (AUC increased from 11.79 $\pm$ 2.73 to 27.91 $\pm$ 1.22 ng·h/mL).

## 2.5 - Long-term treatment with PEGylated lipid nanocapsules in obese/diabetic mice

To investigate the impact of the increased GLP-1 secretion observed after PEGylation on glucose metabolism, a long-term treatment (one month) was performed in diabetic mice (10 weeks HFD), administering exenatide-loaded or unloaded RM LNC PEG (500 $\mu$g/kg) with different administration frequencies (daily or once every other day) (14 weeks of HFD in total, daily or every other day administrations for 4 weeks from week 10). The mice treated less frequently by oral administration (once every other day) (EXE RM LNC PEG; 500 μg/kg), or with identical amounts of unloaded PEGylated nanocapsules (RM LNC PEG), were also compared with those that received non-PEGylated EXE-loaded nanocapsules (EXE RM LNC; 500 μg/kg) following the administration regimen.

The plasma glucose and insulin levels and the calculated HOMA-IR after a 4 weeks of treatment are depicted in **Fig. 18A-C.** The PEGylated groups (loaded or unloaded) administered on a daily basis and the EXE-loaded PEGylated group administered every other day exhibited comparable glucose plasma levels. These levels were in turn comparable to the non-obese/diabetic untreated control mice (p>0.05, **Fig. 18A**). It is noteworthy that only when PEGylated nanocapsules were encapsulating EXE were effective for lowering glucose plasma levels following a long-term treatment. More importantly, they were efficient even with less frequent administration, which was the main goal of the present study (**Fig. 18A**). Additionally, these groups exhibited equivalent insulin resistance, as analyzed by homeostatic model assessment of insulin resistance (HOMA-IR), which were comparable to the control values obtained for the healthy control group (p>0.05) (**Fig. 18C**). Compared with the HFD group, the reduction among these groups was not statistically significant using the Kruskal-Wallis followed by Dunn's post hoc test (**Fig. 18C**). However, significant differences were observed when the Mann-Whitney test was used (p=0.0012 for EXE RM LNC PEG-D versus HFD, p=0.0003 for RM LNC PEG-D treatment versus HFD, and p=0.05 for EXE RM LNC PEG-T treatment versus HFD, respectively).

*2.6 - Conclusion*

In conclusion, increasing GLP-1 secretion and prolonging the antidiabetic effect of the formulations may be achieved via PEGylation of the nanocapsules.

**Example 3: Comparative example**

Shrestha et al. (Nanoscale 2018, see above) provided *in vitro* data on a different lipid-based nanoparticles, as compared to the nanoparticles according to the invention disclosed herein: nanostructured lipid carriers (NLC). Among the differences with lipid nanocapsules, NLC present a solid core and not a liquid core as lipid nanocapsules according to the invention. In Shrestha *et al.*, exenatide and liraglutide were encapsulated and it was demonstrated that these NLC were inducing GLP-1 secretion *in vitro* when presenting a nanoparticle size of 150 nm. Also, in the study of Beloqui et al. (Mol Pharm see above), the GLP-1 secretory effect of NLC (150 nm) was exploited. Among different nanoparticles tested, only NLC presenting a nanoparticle size of 150 nm were able to induce GLP-1 secretion *in vitro.* However, these nanoparticles were not able to induce GLP-1 secretion in further studies *in vivo*, nor decrease hyperglycemia and hyperinsulinemia.

An *in vivo* study was conducted in 4-weeks HFD fed mice (as in example 1). It was administered once orally the NLC encapsulating exenatide (500 μg/kg exenatide dose) before an oral glucose tolerance test (OGTT). As shown in **Fig. 19,** it can be observed no effect on the glucose area under the curve (**Fig. 19A-B**), no increment on the GLP-1 levels (**Fig. 19C-D**), no changes on hyperinsulinemia (**Fig. 19E-F**) and no effect on the insulin resistance index (**Fig. 19G**).

These results demonstrate that two lipid-based nanoparticles, *a priori* presenting a similar composition, do not necessarily induce the same effect.

**Claims**

1. A lipid nanocapsule for oral administration comprising:

    - a solid lipid outer shell; and
    - a lipophilic liquid inner core comprising reverse micelles loaded with one or more incretin mimetics.

2. The lipid nanocapsule according to claim **1,** wherein the solid lipid outer shell comprises one or more surfactants selected in a group comprising ionic surfactants, nonionic surfactants, amphoteric surfactants, lipophilic surfactants, and mixtures thereof.

3. The lipid nanocapsule according to claim **1** or **2,** wherein the lipophilic liquid inner core comprises one or more oils.

4. The lipid nanocapsule according to claim **3,** wherein the one or more oils is a triglyceride, a fatty acid, a fatty acid ester or a mixture thereof.

5. The lipid nanocapsule according to any one of claims **1** to **4,** wherein the reverse micelles comprise one or more ingredients selected in a group comprising a surfactant, an oil and a mixture thereof.

6. The lipid nanocapsule according to any one of claims **1** to **5,** wherein the lipid nanocapsule has a mean diameter ranging from about 100 nm to about 300 nm.

7. The lipid nanocapsule according to any one of claims **1** to **6,** wherein the lipid nanocapsule induces endogenous GLP-1 secretion *in vivo.*

8. The lipid nanocapsule according to any one of claims **1** to **7,** wherein the one or more incretin mimetics is selected in a group comprising albiglutide, dulaglutide, exenatide, liraglutide, lixisenatide and semaglutide, preferably exenatide.

9. The lipid nanocapsule according any one of claims **1** to **8,** wherein it comprises:

  - a solid lipid outer shell comprising a poly-oxyethylene ester of fatty acid, a non-ionic lipophilic surfactant and optionally a PEGylated lipid, in particular a PEGylated phospholipid; and
  - a lipophilic liquid inner core comprising a triglyceride and a fatty acid ester, and comprising reverse micelles loaded with exenatide.

10. The lipid nanocacpsule according to any one of claims **1** to **9,** wherein it comprises:

  - a solid lipid outer shell comprising the surfactants Solutol® HS15, Lipoid® S100 and optionally DSPE-$PEG_{2000}$-$OCH_3$; and
  - a lipophilic liquid inner core comprising the oils Labrafac™ Lipophile WL1349 and/or Plurol® CC 497 or Peceol™, and comprising reverse micelles loaded with exenatide.

11. A pharmaceutical composition comprising:

  - a therapeutically effective amount of a lipid nanocapsule for oral administration comprising a solid lipid outer shell and a lipophilic liquid inner core comprising reverse micelles loaded with one or more incretin mimetics, and
  - a pharmaceutically acceptable vehicle.

12. A medicament comprising a therapeutically effective amount of a lipid nanocapsule for oral administration comprising a solid lipid outer shell and a lipophilic liquid inner core comprising reverse micelles loaded with one or more incretin mimetics.

13. A lipid nanocapsule comprising a solid lipid outer shell and a lipophilic liquid inner core comprising reverse micelles loaded with one or more incretin mimetics for use in treating and/or preventing a disorder associated with a GLP-1 dysfunction in a subject in need thereof.

14. The lipid nanocapsule for use according to claim **13,** wherein said disorder is selected in a group comprising type 2 diabetes mellitus (T2DM), obesity, inflammatory bowel disease (IBD), pancreatitis, dyslipidemia, non-alcoholic fatty liver disease, hyperglycemia, liver steatosis, overweight, nonalcoholic fatty liver disease (NAFLD), nonalcoholic steatohepatitis (NASH), insulin resistance, hyperinsulinemia, glucose intolerance, hyperglycemia, metabolic syndrome, prediabetes, impaired fasting glucose, hyperphagia, altered food intake behaviour, hepatic insulin resistance, whole body insulin resistance, accelerated transit, adipose tissue inflammation, cardiac dysfunctions, acute myocardial infarction, hypertension, cardiovascular disease, atherosclerosis, peripheral arterial disease, stroke, heart failure, coronary heart disease, kidney disease, diabetic complications, neuropathy, and gastroparesis, preferably selected in a group comprising type 2 diabetes mellitus (T2DM), obesity and inflammatory bowel disease (IBD).

15. A kit for treating and/or preventing a disorder associated with a GLP-1 dysfunction comprising:

  - one or more lipid nanocapsules comprising a solid lipid outer shell and a lipophilic liquid inner core comprising reverse micelles loaded with one or more incretin mimetics; and
  - one or more oral hypoglycemic agent.

**Patentansprüche**

1. Lipid-Nanokapsel zur oralen Verabreichung, umfassend:

- eine feste Lipid-Außenhülle; und
- einen lipophilen flüssigen inneren Kern, der mit einem oder mehreren Inkretin-Mimetika beladene umgekehrte Mizellen umfasst.

2. Lipid-Nanokapsel nach Anspruch **1,** wobei die feste Lipid-Außenhülle ein oder mehrere Tenside umfasst, die aus einer Gruppe ausgewählt sind, die ionische Tenside, nichtionische Tenside, amphotere Tenside, lipophile Tenside und Mischungen davon umfasst.

3. Lipid-Nanokapsel nach Anspruch **1** oder **2,** wobei der lipophile flüssige innere Kern ein oder mehrere Öle umfasst.

4. Lipid-Nanokapsel nach Anspruch **3,** wobei das eine oder die mehreren Öle ein Triglycerid, eine Fettsäure, ein Fettsäureester oder eine Mischung davon sind.

5. Lipid-Nanokapsel nach einem der Ansprüche **1** bis **4,** wobei die umgekehrten Mizellen einen oder mehrere Inhaltsstoffe umfassen, die aus einer Gruppe ausgewählt sind, die ein Tensid, ein Öl und eine Mischung davon umfasst.

6. Lipid-Nanokapsel nach einem der Ansprüche **1** bis **5,** wobei die Lipid-Nanokapsel einen mittleren Durchmesser von etwa 100 nm bis etwa 300 nm aufweist.

7. Lipid-Nanokapsel nach einem der Ansprüche **1** bis **6,** wobei die Lipid-Nanokapsel *in vivo* eine endogene GLP-1-Sekretion induziert.

8. Lipid-Nanokapsel nach einem der Ansprüche **1** bis **7,** wobei das eine oder die mehreren Inkretin-Mimetika aus einer Gruppe ausgewählt sind, die Albiglutid, Dulaglutid, Exenatid, Liraglutid, Lixisenatid und Semaglutid, vorzugsweise Exenatid, umfasst.

9. Lipid-Nanokapsel nach einem der Ansprüche **1** bis **8,** wobei sie Folgendes umfasst:

- eine feste Lipid-Außenhülle, die einen Polyoxyethylenester von Fettsäure, ein nichtionisches lipophiles Tensid und optional ein PEGyliertes Lipid, insbesondere ein PEGyliertes Phospholipid, umfasst; und
- einen lipophilen flüssigen inneren Kern, der ein Triglycerid und einen Fettsäureester umfasst und mit Exenatid beladene umgekehrte Mizellen umfasst.

10. Lipid-Nanokapsel nach einem der Ansprüche **1** bis **9,** wobei sie Folgendes umfasst:

- eine feste Lipid-Außenhülle, die die Tenside Solutol® HS15, Lipoid® S100 und optional DSPE-PEG$_{2000}$-OCH$_3$ umfasst; und
- einen lipophilen flüssigen inneren Kern, der die Öle Labrafac™ Lipophile WL1349 und/oder Plurol® CC 497 oder Peceol™ umfasst und mit Exenatid beladene umgekehrte Mizellen umfasst.

11. Pharmazeutische Zusammensetzung, umfassend:

- eine therapeutisch wirksame Menge einer Lipid-Nanokapsel zur oralen Verabreichung, die eine feste Lipid-Außenhülle und einen lipophilen flüssigen inneren Kern umfasst, der mit einem oder mehreren Inkretin-Mimetika beladene umgekehrte Mizellen umfasst, und
- einen pharmazeutisch akzeptablen Träger.

12. Arzneimittel, das eine therapeutisch wirksame Menge einer Lipid-Nanokapsel zur oralen Verabreichung umfasst, die eine festen Lipid-Außenhülle und einen lipophilen flüssigen inneren Kern umfasst, der mit einem oder mehreren Inkretin-Mimetika beladene umgekehrte Mizellen umfasst.

13. Lipid-Nanokapsel, die eine feste Lipid-Außenhülle und einen lipophilen flüssigen inneren Kern umfasst, der mit einem oder mehreren Inkretin-Mimetika beladene umgekehrte Mizellen umfasst, zur Verwendung bei der Behandlung und/oder Prävention von Störungen im Zusammenhang mit einer GLP-1-Dysfunktion bei einem Patienten, der dies benötigt.

14. Lipid-Nanokapsel zur Verwendung nach Anspruch **13,** wobei die Störung aus einer Gruppe ausgewählt ist, die Typ-2-Diabetes mellitus (T2DM), Adipositas, entzündliche Darmerkrankung (IBD), Pankreatitis, Dyslipidämie, nichtalkoholische Fettlebererkrankung, Hyperglykämie, Lebersteatose, Übergewicht, nichtalkoholische Fettlebererkrankung (NAFLD), nichtalkoholische Steatohepatitis (NASH), Insulinresistenz, Hyperinsulinämie, Glukoseintoleranz, Hyperglykämie, metabolisches Syndrom, Prädiabetes, gestörte Nüchternglukose, Hyperphagie, verändertes Nahrungsaufnahmeverhalten, hepatische Insulinresistenz, Insulinresistenz des ganzen Körpers, beschleunigter Transit, Entzündung des Fettgewebes, Herzfunktionsstörungen, akuter Myokardinfarkt, Hypertonie, Herz-Kreislauf-Erkrankungen, Arteriosklerose, periphere arterielle Verschlusserkrankung, Schlaganfall, Herzinsuffizienz, koronare Herzkrankheit, Nierenerkrankungen, diabetische Komplikationen, Neuropathie und Gastroparese umfasst,, vorzugsweise ausgewählt aus einer Gruppe, die Typ-2-Diabetes mellitus (T2DM), Adipositas und entzündliche Darmerkrankung (IBD) umfasst.

15. Kit zur Behandlung und/oder Prävention einer Störung im Zusammenhang mit einer GLP-1-Dysfunktion, umfassend:

- eine oder mehrere Lipid-Nanokapseln, die eine feste Lipid-Außenhülle und einen lipophilen flüssigen inneren Kern umfassen, der mit einem oder mehreren Inkretin-Mimetika beladene umgekehrte Mizellen umfasst; und
- ein oder mehrere orale Hypoglykämika.

## Revendications

1. Une nanocapsule lipidique pour administration orale comprenant :

- une enveloppe externe lipidique solide ; et
- un noyau interne liquide lipophile comprenant des micelles inversées chargées d'un ou plusieurs mimétiques de l'incrétine.

2. La nanocapsule lipidique selon la revendication **1,** dans laquelle l'enveloppe externe lipidique solide comprend un ou plusieurs tensioactifs choisis dans un groupe comprenant des tensioactifs ioniques, des tensioactifs non ioniques, des tensioactifs amphotères, des tensioactifs lipophiles et des mélanges de ceux-ci.

3. La nanocapsule lipidique selon la revendication **1** ou **2,** dans laquelle le noyau interne liquide lipophile comprend une ou plusieurs huiles.

4. La nanocapsule lipidique selon la revendication **3,** dans laquelle la ou les huiles est un triglycéride, un acide gras, un ester d'acide gras ou un mélange de ceux-ci.

5. La nanocapsule lipidique selon l'une quelconque des revendications **1** à **4,** dans laquelle les micelles inversées comprennent un ou plusieurs ingrédients choisis dans un groupe comprenant un tensioactif, une huile et un mélange de ceux-ci.

6. La nanocapsule lipidique selon l'une quelconque des revendications **1** à **5,** dans laquelle la nanocapsule lipidique a un diamètre moyen allant d'environ 100 nm à environ 300 nm.

7. La nanocapsule lipidique selon l'une quelconque des revendications **1** à **6,** dans laquelle la nanocapsule lipidique induit la sécrétion endogène de GLP-1 *in vivo.*

8. La nanocapsule lipidique selon l'une quelconque des revendications **1** à **7,** dans laquelle le ou les mimétiques de l'incrétine est choisi dans un groupe comprenant l'albiglutide, le dulaglutide, l'exénatide, le liraglutide, le lixisénatide et le sémaglutide, de préférence l'exénatide.

9. La nanocapsule lipidique selon l'une quelconque des revendications **1** à **8,** dans laquelle elle comprend :

- une enveloppe externe lipidique solide comprenant un ester poly-oxyéthylène d'acide gras, un tensioactif lipophile non ionique et optionnellement un lipide PEGylé, en particulier un phospholipide PEGylé ; et
- un noyau interne liquide lipophile comprenant un triglycéride et un ester d'acide gras, et comprenant des micelles inversées chargées d'exénatide.

10. La nanocapsule lipidique selon l'une quelconque des revendications **1** à **9,** dans laquelle elle comprend :

- une enveloppe externe lipidique solide comprenant les tensioactifs Solutol® HS15, Lipoid® S100 et optionnellement DSPE-PEG$_{2000}$-OCH3 ; et
- un noyau interne liquide lipophile comprenant les huiles Labrafac™ Lipophile WL1349 et/ou Plurol®CC 497 ou Peceol™, et comprenant des micelles inversées chargées d'exénatide.

11. Une composition pharmaceutique comprenant :

- une quantité thérapeutiquement efficace d'une nanocapsule lipidique pour administration orale comprenant une enveloppe externe lipidique solide et un noyau interne liquide lipophile comprenant des micelles inversées chargées d'un ou plusieurs mimétiques de l'incrétine, et
- un véhicule pharmaceutiquement acceptable.

12. Un médicament comprenant une quantité thérapeutiquement efficace d'une nanocapsule lipidique pour administration orale comprenant une enveloppe externe lipidique solide et un noyau interne liquide lipophile comprenant des micelles inversées chargées d'un ou plusieurs mimétiques de l'incrétine.

13. Une nanocapsule lipidique comprenant une enveloppe externe lipidique solide et un noyau interne liquide lipophile comprenant des micelles inversées chargées d'un ou plusieurs mimétiques de l'incrétine pour son utilisation dans le traitement et/ou la prévention d'un trouble associé à un dysfonctionnement du GLP-1 chez un sujet qui en a besoin.

14. La nanocapsule lipidique pour son utilisation selon la revendication **13,** dans laquelle ledit trouble est choisi dans un groupe comprenant le diabète sucré de type 2 (T2DM), obésité, maladie inflammatoire chronique de l'intestin (IBD), pancréatite, dyslipidémie, stéatose hépatique non alcoolique, hyperglycémie, stéatose hépatique, surpoids, stéatose hépatique non alcoolique (NAFLD), stéatohépatite non alcoolique (NASH), résistance à l'insuline, hyperinsulinémie, intolérance au glucose, hyperglycémie, syndrome métabolique, prédiabète, hyperglycémie modérée à jeun, hyperphagie, modification du comportement alimentaire, résistance hépatique à l'insuline, résistance à l'insuline dans tout le corps, transit accéléré, inflammation du tissu adipeux, dysfonctionnements cardiaques, infarctus aigu du myocarde, hypertension, maladie cardiovasculaire, athérosclérose, maladie artérielle périphérique, accident vasculaire cérébral, insuffisance cardiaque, maladie coronarienne, maladie rénale, complications diabétiques, neuropathie et gastroparésie, de préférence choisi dans un groupe comprenant le diabète sucré de type 2 (T2DM), l'obésité et une maladie inflammatoire chronique de l'intestin (IBD).

15. Un kit pour traiter et/ou prévenir un trouble associé à un dysfonctionnement du GLP-1, comprenant :

- une ou plusieurs nanocapsules lipidiques comprenant une enveloppe externe lipidique solide et un noyau interne liquide lipophile comprenant des micelles inversées chargées d'un ou plusieurs mimétiques de l'incrétine ; et
- un ou plusieurs agents hypoglycémiants oraux.

FIG. 1A-F

**FIG. 2A-B**

FIG. 3

**FIG. 4A-D**

**FIG. 5**

**FIG. 6**

**FIG. 7**

FIG. 8

**FIG. 9A-B**

**FIG. 10A-B**

**FIG. 11**

FIG. 12

FIG. 13A

**FIG. 13B-C**

**FIG. 14A-B**

**FIG. 14C-E**

**(a)**

FIG. 15A

**(b)**

FIG. 15B

**FIG. 16**

**Fig. 17A**

FIG. 17B-C

FIG. 17D-E

FIG. 17F

**FIG. 18A**

**FIG. 18B-C**

FIG. 19A

FIG. 19B-C

FIG. 19D-E

FIG. 19F-G

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017087096 A1
- US 2017087096 A
- WO 2018157202 A

**Non-patent literature cited in the description**

- **BURANT**. *Diabetes Care*, 2013, vol. 36 (2), S175-179
- **SHERESTHA et al.** *Nanoscale*, 2018, vol. 10 (2), 603-613
- **ANTON et al.** Int. J. of Pharmaceutics. Elsevier, 2010, vol. 398, 204-209
- **SHRESTHA et al.** *Nanoscale*, 2018, vol. 10, 603-613
- **BELOQUI et al.** *Mol Pharm.*, 2016, vol. 13, 4222-4230
- **AMRUTKAR et al.** *Diabetes*, 2015, vol. 64, 2791-2804
- **HEURTAULT et al.** *Pharm Res.*, 2002, vol. 19, 875-880
- **BELOQUI et al.** *Journal of Controlled Release*, 2013, vol. 166, 115-123
- **XU et al.** *Mol Pharm.*, 2018, vol. 15, 108-115
- **ZHANG et al.** *Comput Methods Programs Biomed.*, 2010, vol. 99, 306-314
- *Biol Chem.*, 1957, vol. 226, 497-509
- **EVERARD et al.** *Nat Commun.*, 2019, vol. 10, 457
- **ANTON et al.** *Int J Pharm.*, 2010, vol. 398, 204-209
- **ROGER et al.** *Int J Pharm.*, 2009, vol. 379, 260-265
- **PERSEGHIN**. *Diabetes Care.*, 2011, vol. 34 (2), 367-370
- **LEBOVITZ ; BANERJI**. *Diabetes Care*, 2005, vol. 28, 2322-2325
- **WEISBERG et al.** *Clin Invest.*, 2003, vol. 112, 1796-1808
- **HERNANDEZ et al.** *Cell Metab.*, 2014, vol. 20, 499-511
- **CANI et al.** *Gut*, 2009, vol. 58, 1091-1103
- **WANG et al.** *Bio-protocol.*, 2017, vol. 7, e2394
- **SHRESTHA et al.** *Nanoscale*, 2018
- **BELOQUI et al.** *Mol Pharm*